# EUROPEAN PATENT SPECIFICATION

(11) **EP 2 120 974 B1**
(45) Date of publication and mention of the grant of the patent: **01.03.2017**
(21) Application number: 07716870.6
(22) Date of filing: 22.01.2007
(51) Int. Cl.: A61K 47/54, A61K 47/55, A61K 47/64, A61K 47/68, A61K 47/69, A61K 31/795, C07K 7/08, A61P 31/14, A61P 31/16, C07K 7/06

(54) **SELF-ASSEMBLING AMPHIPHILIC POLYMERS AS ANTIVIRAL AGENTS**
SELBST-ZUSAMMENBAUENDE AMPHIPHILE POLYMERE ALS ANTIVIRALE MITTEL
POLYMÈRES AMPHIPHILES À AUTO-ASSEMBLAGE EN TANT QU'AGENTS ANTIVIRAUX

(43) Date of publication of application: 25.11.2009
(73) Proprietor: Allexcel, Inc., West Haven, CT 06516 (US)
(72) Inventor: ONTON, Ann, Louise, Fairfield, CT 06825 (US); DIWAN, Anil, West Haven, CT 06516 (US); TATAKE, Jayant, G., Sandy Hook, CT 06482 (US)
(74) Representative: Viering, Jentschura & Partner mbB Patent- und Rechtsanwälte
(86) International application number: PCT/US2007/001607
(87) International publication number: WO 2008/091246

(56) References cited:
- WO-A1-98/26662
- WO-A1-2007/084126
- WO-A2-2006/034081
- THOMAS L. LENTZ: "Rabies virus binding to an acetylcholine receptor [alpha]-subunit peptide", JOURNAL OF MOLECULAR RECOGNITION, vol. 3, no. 2, 1 April 1990 (1990-04-01), pages 82-88, XP55041338, ISSN: 0952-3499, DOI: 10.1002/jmr.300030205
- PAUL T. WILSON ET AL: "Binding of .alpha.-bungarotoxin to synthetic peptides corresponding to residues 173-204 of the .alpha. subunit of Torpedo, calf, and human acetylcholine receptor and restoration of high-affinity binding by sodium dodecyl sulfate", BIOCHEMISTRY, vol. 27, no. 18, 1 September 1988 (1988-09-01), pages 6667-6674, XP55040914, ISSN: 0006-2960, DOI: 10.1021/bi00418a004
- DONNELLY-ROBERTS D L ET AL: "Antibodies against an alpha-bungarotoxin-binding peptide of the alpha-subunit of the acetylcholine receptor", BIOCHEMICAL AND BIOPHYSICAL RESEARCH COMMUNICATIONS, ACADEMIC PRESS INC. ORLANDO, FL, US, vol. 160, no. 1, 14 April 1989 (1989-04-14), pages 289-295, XP024772786, ISSN: 0006-291X, DOI: 10.1016/0006-291X(89)91654-9 [retrieved on 1989-04-14]
- MOCHALOVA L V ET AL: "Synthetic polymeric inhibitors of influenza virus receptor-binding activity suppress virus replication", ANTIVIRAL RESEARCH, ELSEVIER BV, NL, vol. 23, no. 3-4, 1 April 1994 (1994-04-01), pages 179-190, XP023702847, ISSN: 0166-3542, DOI: 10.1016/0166-3542(94)90016-7 [retrieved on 1994-04-01]
- REUTER J D ET AL: "INHIBITION OF VIRAL ADHESION AND INFECTION BY SIALIC-ACID- CONJUGATED DENDRITIC POLYMERS", BIOCONJUGATE CHEMISTRY, ACS, WASHINGTON, DC, US, vol. 10, no. 2, 1 March 1999 (1999-03-01), pages 271-278, XP000804253, ISSN: 1043-1802, DOI: 10.1021/BC980099N
- CHOI S-K ET AL: "GENERATION AND IN SITU EVALUATION OF LIBRARIES OF POLY(ACRYLIC ACID) PRESENTING SIALOSIDES AS SIDE CHAINS AS POLYVALENT INHIBITORS OF INFLUENZA-MEDIATED HEMAGGLUTINATION", JOURNAL OF THE AMERICAN CHEMICAL SOCIETY, ACS PUBLICATIONS, US, vol. 119, no. 18, 7 May 1997 (1997-05-07), pages 4103-4111, XP002068481, ISSN: 0002-7863, DOI: 10.1021/JA963519X
- PIETROPAOLO ET AL: "Effect of natural and semisynthetic polymers on rabies virus infection in CER cells", RESEARCH IN VIROLOGY, ELSEVIER, PARIS, FR, vol. 144, 1 January 1993 (1993-01-01), pages 151-158, XP022352664, ISSN: 0923-2516, DOI: 10.1016/S0923-2516(06)80023-3
- ITOH M. ET AL.: 'Suppression of Influenza Virus Infection by an N-Thioacetylneuramine Acid Acrylamide Copolymer Resistant to Neuraminidase' VIROLOGY vol. 212, 1995, pages 340 - 347, XP002036922
- REUTER J.D. ET AL.: 'Inhibition of Viral Adhesion and Infection by Sialic-Acid-Conjugated Dendritic Polymers' BIOCONJUGATE CHEMISTRY vol. 10, 1999, pages 271 - 278, XP000804253
- MYUNG GI-BAEK ET AL.: 'Design and Synthesis of Novel Glycopolythiophene Assemblies for Colorimetric Detection of Influenza Virus and E. coli' BIOCONJUGATE CHEMISTRY vol. 11, 2000, pages 777 - 788, XP008110959

## Description

### FIELD OF THE INVENTION

The present invention relates to the fields of amphiphilic polymers, and specifically to biocompatible micelle-forming comb-type polymers. The invention also relates to the fields of targeted drug delivery and antiviral agents.

### BACKGROUND

Amphiphilic block copolymers comprising a hydrophobic block and a hydrophilic block have been well studied in recent years, because of their capacity for self-assembly into a variety of nanostructures as the surrounding solvent is varied. See Cameron et al., Can. J. Chem./Rev. Can. Chim. 77:1311-1326 (1999). In aqueous solutions, the hydrophobic compartment of an amphiphilic polymer has a tendency to self-assemble in order to avoid contact with water and to minimize the free interfacial energy of the system. At the same time, the hydrophilic blocks form a hydrated "corona" in the aqueous environment, and so the aggregates maintain a thermodynamically stable structure. The result is a stable, latex-like colloidal suspension of polymer aggregate particles having hydrophobic cores and hydrophilic coronas.

Comb-type amphiphilic co-polymers differ from block co-polymers in that the backbone is largely hydrophobic or hydrophilic, with polymer chains of opposite polarity pendant from the backbone rather than incorporated into it. Comb-type copolymers have been prepared with hydrophobic backbones and hydrophilic branches (Mayes et al., US Patent No. 6,399,700), and also with hydrophilic backbones and hydrophobic branches (Watterson et al., U.S. Patent No. 6,521,736). The former were used to provide multivalent presentation of ligands for cell surface receptors, while the latter were used to solubilize drugs and deliver them to cells.

Amphiphilic polymer aggregates have been studied as carriers for solubilizing insoluble drugs, targeted drug delivery vehicles, and gene delivery systems. They have a more stable structure than conventional low-molecular-weight micelles, due to chain entanglement and/or the crystallinity of the interior hydrophobic region. The polymeric nature of the vehicle renders the aggregates relatively immune to the disintegration that ordinary liposomes suffer when diluted below their critical micelle concentration. The absence of a bilayer membrane enables them to more readily fuse with cell membranes and deliver their payload directly to the cell. The amphiphilic nature of the aggregates also confers detergent-like activity, and appropriately targeted aggregates appear to be capable fusing with and disrupting viral coat proteins.

Due to the excellent biocompatibility poly(ethylene glycol) (PEG), and the apparent ability of PEG-coated "stealth" particles to evade the reticuloendothelial system, micelles, liposomes, and polymers incorporating PEG have been extensively considered as materials for drug delivery systems. There are many reports of the use of poly(ethylene glycol) (PEG) as the hydrophilic component of PEG-lipids (forming liposomes and micelles); see for example Krishnadas et al., Pharm. Res. 20:297-302 (2003). Self-assembling amphiphilic block copolymers, which self-assemble into the more robust "polymersomes", have also been investigated as vehicles for drug solubilization and delivery (Photos et al, J. Controlled Release, 90:323-334 (2003)). See also Gref et al., Int. Symp. Controlled Release Mater. 20:131 (1993); Kwon et al., Langmuir, 9:945 (1993); Kabanov et al., J. Controlled Release, 22:141 (1992); Allen et al., J. Controlled Release, 63:275 (2000); Inoue et al., J. Controlled Release, 51:221 (1998); Yu and Eisenberg, Macromolecules, 29:6359 (1996); Discher et al., Science, 284:113 (1999); Kim et al., U.S. Patent No. 6,322,805; Seo et al., U.S. Patent No. 6,616,941 and *Seo et al.,* European Patent No. EP 0583955. The use of poly(ethyleneimine) (PEI) in this capacity has also been reported, with a focus on delivery of oligonucleotides (Nam et al., U.S. Patent No. 6,569,528; Wagner et al., U.S. Patent application publication No. 20040248842). In a similar vein, Luo et al., in Macromolecules 35:3456 (2002), describe PEG-conjugated polyamidoamine ("PAMAM") dendrimers suitable for delivery of polynucleotides.

In addition to the need to solubilize, distribute, and deliver drugs, there is a need for targeted drug delivery systems that home in specifically on a target tissue, tumor, or organ. This is usually accomplished by attachment of antibodies or other ligands with a specific affinity for cell walls at the target site. However, PEG lacks functional groups except at the ends of the polymer chains, and the majority of the terminal groups are inevitably taken up by bonds to the other block copolymer component. For this reason, attachment of targeting moieties such as antibodies or cell-adhesion molecules to PEG block copolymers is generally limited to the non-PEG block, which unfortunately is not the part of the copolymer that is normally exposed in the corona of the self-assembled aggregate.

The phase separation phenomenon which results in the self-assembly of block copolymers into polymer aggregates is readily reversible, and attempts have been made to increase the stability of the aggregates by cross-linking the hydrophobic core (see European Patent No. EP 0552802). Covalent attachment of the drug to the hydrophobic component of a block copolymer has also been attempted (Park and Yoo, U.S. Patent No. 6,623,729; European Patent No. EP 0397307).

Dendritic polymers are readily conjugated to targeting moieties, and also have the potential to target specific cells in vivo (Singh et al. (1994) Clin. Chem. 40:1845) and block adhesion of viral and bacterial pathogens to biological substrates. Comb-branched and dendrigraft polymers conjugated to multiple sialic acid have been evaluated for their ability to inhibit virus hemagglutination and to block infection of mammalian cells in vitro (Reuter et al. (1999) Bioconjugate Chem. 10:271). The most effective virus inhibitors were the comb-branched and dendrigraft macromolecules, which showed up to 50,000-fold increased activity against these viruses.

Recently, the pharmaceutical company Starpharma announced the successful development of a dendrimer-based biocide (VivaGel™) that prevents HIV infection by binding to receptors on the virus's surface (Halford (2005) Chem. & Eng. News 83 (24):30). Chen at al. (2000) (Biomacromolecules. 1:473) have reported that quaternary ammonium functionalized poly(propyleneimine) dendrimers are very potent biocides.

There remains a need for a drug delivery system that is stable, biocompatible, amenable to the attachment of targeting moieties to the exterior of the aggregates, and efficient at delivering drugs to the desired cellular targets. There is also a need for targeted antiviral agents that are similarly stable and biocompatible.

### SUMMARY OF THE INVENTION

The present invention provides biocompatible comb-type polymer molecules, comprising a hydrophilic backbone having branch-point moieties, and hydrophobic branches attached at these branch-point moieties. The invention provides aqueous suspensions of polymer aggregates formed from such polymers, and provides methods for solubilizing insoluble or sparingly-soluble organic compounds, such as drugs, dyes, vitamins, and the like, by incorporating such compounds in the hydrophobic cores of the polymer aggregates. The method for solubilizing a water-insoluble organic species in an aqueous solvent basically comprises contacting the water-insoluble organic species with a polymer of the invention in an aqueous or mixed-aqueous solvent.

The invention also provides comb-type polymers for use in a a method for the treatment or prevention of an infection of an animal by a virus, which comprises administering to said animal a comb-type polymer consisting essentially of the following structure:

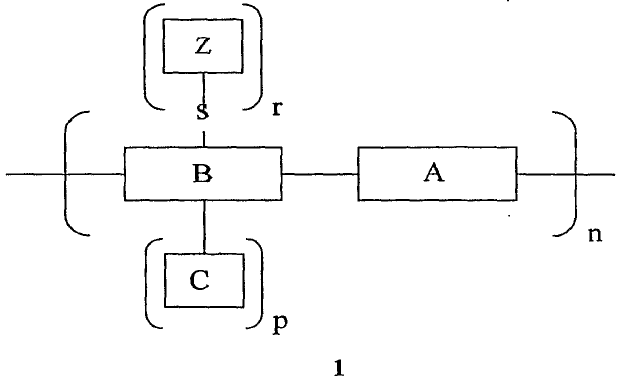

The structure comprises a backbone formed of alternating branch-point moieties B and hydrophilic, water-soluble polymer blocks A. Hydrophobic side chains C and ligands Z are attached to the branch-point moieties. Preferably, the side chains C are linear or branched hydrocarbons, optionally substituted with one or more hydrophilic substituents, or C₆-C₃₀ cyclic or polycyclic hydrocarbons optionally substituted with one or more hydrophilic substituents. Side chains C may also be hydrophobic amino acids, peptides, or polymers. Suitable hydrophilic substituents for the side chains C are hydroxyl, carboxy, and amino groups, as well as amide, sulfonamide, sulfoxide and sulfone groups. Preferred hydrophilic substituents are polar aprotic groups such as tertiary amide, sulfoxide, and sulfone.

The ligands Z are ligands having specific binding affinity for the surface of a virus. "Specific binding affinity" means that the ligand is capable of binding to the surface of a virus in vivo in the presence of the many cellular surfaces and macromolecules found in mammalian body. The group s is a bond or a spacer moiety, and when s is a spacer each s may carry from 1 to 4 groups Z. The value of n ranges from 3 to about 100; the average value of p ranges from 1 to 2, and the average value of r ranges from 1 to 4.

The branch point moiety B is a multi-valent moiety having bonds to two polymer blocks A, bonds to 1-2 side chains C (on average), and one or more bonds to spacers "s" and/or ligands Z. In particular embodiments, the bonds to B and s and/or Z are established via a plurality of reactive functional groups, which are capable of serving as attachment points. In particularly preferred embodiments, targeting moieties such as ligands or antibodies are covalently attached to the branch-point moieties of the polymers of the invention, and a drug is incorporated into the core of the aggregates, so as to form a targeted drug complex.

The invention also provides biocompatible comb-type polymer molecules as described above, which even in the absence of a small-molecule therapeutic have inherent antiviral properties. This antiviral activity is thought to be due to the detergent-like ability of the amphiphilic polymers to disrupt the outer coating of virus particles. In preferred embodiments, the antiviral activity is enhanced by the attachment of targeting moieties having binding affinity for the surface of the targeted virion.

The invention further provides methods for the preparation of the comb-type polymers, aggregates, and targeted polymer aggregates and drug complexes described herein. The polymers of the invention self-assemble into polymer aggregates that efficiently solubilize, distribute, and deliver drugs in vivo; have antiviral activity; are non-toxic, biocompatible, and stable; and are capable of bearing multiple cell- and virus-targeting moieties on their exterior surfaces.

### BRIEF DESCRIPTION OF THE DRAWINGS

Fig. 1 shows the effect of administration of a composition of the invention on the mean survival time of influenza-infected mice.
Fig. 2 shows the increase in survival time of influenza-infected mice when treated with a composition of the invention.
Fig. 3 shows the weight loss over the course of 7 days of influenza-infected mice when treated with a composition of the invention.

### DETAILED DESCRIPTION OF THE INVENTION

The polymers of the invention, referred to herein as "π-polymers", have a comb-type architecture, with a backbone formed of alternating branch-point moieties B and hydrophilic, water-soluble polymer blocks A; and having a plurality of hydrophobic side chains C attached to each branch-point moiety, as shown in Formula 1. The side chains C are relatively short, hydrophobic moieties, which may be aliphatic molecules, chains or oligomers. The value of p is ideally an integer, either 2, 3, or 4. In practice the side chains are most often introduced with less-than-perfect efficiency via chemical reactions, resulting in an average value of p for the polymer preparation as a whole that is not the intended integer. Non-integer average values can also be obtained by design, as discussed below. Thus, the average value of p in the polymers of the invention is greater than one and may be as high as four (1 < p ≤ 4). In preferred embodiments, p ranges from about 2 to 4, and most preferably 1.5 < p ≤ 2.

The backbone polymer block A is selected from poly(ethylene glycol), polypropylene glycol), poly(ethylene imine), poly(vinyl alcohol), and poly(vinylpyrrolidone). Preferably, the polymer units A are poly(ethylene glycol) chains of formula-(CH₂CH₂O)ₘ- where m is between 1 and 10,000, preferably between 3 and 3,000.

In the manufacture of poly(ethylene glycol) of various grades, it is known in the industry to couple a divalent linker moiety (*e*.*g*., bisphenol A diglycidyl ether) to two poly(ethylene glycol) chains, effectively doubling the molecular weight of the polymer while retaining a relatively narrow molecular weight range. The resulting "poly(ethylene glycol)" molecules are consequently interrupted at the midpoint of the polymer chain by the non-glycol linker moiety (see, *e.g.,* the poly(ethylene glycol)-bisphenol A diglycidyl ether adduct, CAS registry No. 37225-26-6). Higher oligomers, *i.e.* those having three PEG chains separated by two bisphenol A diglycidyl ether moieties, are also known, see for example international patent application WO 00/24008. As used herein, therefore, the terms "poly(ethylene glycol)" and "poly(propylene glycol)" encompass poly(ethylene glycol) and poly(propylene glycol) polymer chains that incorporate non-glycol linker units, including bisphenol A diglycidyl ether, bisphenol B diglycidyl ether, bisphenol S diglycidyl ether, and hydroquinone diglycidyl ether. For purposes of this specification, any such linker moieties are not counted as "monomer units".

The polymer block A most preferably has an average length of between twenty and fifty monomer units. The polyethylene glycol chains may be end-substituted with functional groups suitable for use as linkers to other moieties, including amino, mercapto, acrylate, acrylamide, maleate, maleimide, at one or both ends. The value of n ranges from 1 to 1000 and is preferably between 3 and 100. The overall molecular weight of the π-polymer may range from 1000 to 100,000 daltons or more; it is preferably above 2,000 daltons, and more preferably above 7,000 daltons.

Hydrophobic moieties C may be the same or different, and may be for example linear hydrocarbons (optionally substituted with one or more hydrophilic substituents), polycyclic hydrocarbons (optionally substituted with one or more hydrophilic substituents), hydrophobic amino acids, peptides and polymers. Suitable hydrophilic substituents include, hydroxyl, ether, cyano, and amide functional groups. Specifically contemplated are C₈ to C₂₀ alkyl groups bearing ω-hydroxyl, ω-cyano, ω-amido, or ω-alkoxy substituents. In this context, the term "substituent" includes the substitution of a heteroatom, such as O, N, or S, for a carbon atom in the hydrocarbon chain or ring system of the moiety C. Thus, ether and amide linkages, and heterocyclic rings, may be incorporated into C.

Hydrophobic moieties C are preferably relatively short (C₈-C₂₀) aliphatic chains, but may also be short oligomers. Suitable oligomers include oligo hydroxy acids such as poly(glycolic acid), poly(DL-lactic acid), poly(L-lactic acid), and copolymers of poly(glycolic acid) and poly(lactic acid)hydroxy acids, and poly(amino acids), poly(anhydrides), poly(orthoesters), and poly(phosphoesters), polylactones such as poly(epsilon-caprolactone) poly(delta-valerolactone) poly(gamma-butyrolactone) and poly(beta-hydroxybutyrate). C moieties may also be selected from hydrophobic molecules, such as cholesterol, cholic acid, lithocholic acid, and hydrophobic peptides. The molecular weight of each moiety C is greater than 40, preferably between 50 and 1,000, and most preferably between 100 and 500. The logP value (octanol-water) of the molecule C-H is greater than about 1.4, and preferably greater than about 2.0, and more preferably greater than about 2.5. In general, any moiety C is thought to be suitable for use in the present invention if the molecule C-H is substantially insoluble in water. "Substantially insoluble" means that liquid C-H forms a separate phase when mixed with water.

It is a distinguishing feature of the comb polymers of this invention that the side chains C are not regularly and uniformly distributed along the polymer chain, but rather occur in clusters [C]p. These clusters are spaced more or less regularly along the polymer chain, depending on the degree of monodispersity of the polymer units A. Thus, the distance between two side chains C attached to a common branching moiety B is different from the distance between two side chains attached to different branching moieties.

Also disclosed herein are comb-polymers, wherein the branch-point moieties B further comprise one or more reactive functional groups X, as shown in Formula 2.

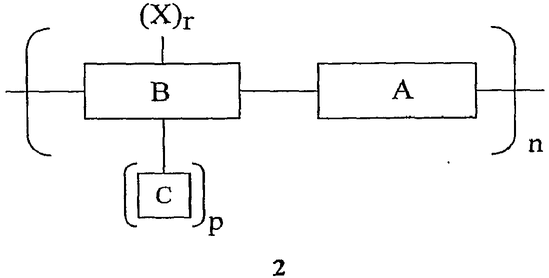

In Formula **2,** the individual reactive groups X may be the same or may be different from one another, and may optionally be blocked or protected as may be necessary during assembly of the polymer **2.** The average value of r will range from 0 (no X groups) to about 4. Typically, the reactive groups will be selected from functional groups known in the art to be useful for forming covalent linkages between molecular species. The groups X serve as attachment points for drug molecules, tissue- or cell-targeting moieties, virus-targeting moieties, or matrix attachment moieties (such as for the purpose of coating the surface of a stent or other medical device). In certain embodiments, there may be a single attachment point X. In other embodiments, there may be three or four different types of reactive groups. The matrix attachment moiety may attach to a matrix via covalent bonds, specific noncovalent interactions (e.g., antibody-antigen, or non-specific interactions (e.g., via ionic pairing or "hydrophobic" interaction). Suitable reactive groups X include -OH, -NH₂, -SH, -CHO, -NHNH₂, -COOH, -CONHNH₂, haloacyl, acetoacetyl, -CN, -OCN, -SCN, -NCO, -NCS, reactive double bonds such as vinylic, acrylic, allylic, maleic, cinnamic, and the like, and groups with reactive triple bonds such as acetylenecarboxy and acetylenecarboxamido (suitable for Michael additions, Diels-Alder reactions, and free radical addition reactions). -

Cell-targeting moieties include receptor-specific ligands, antibodies, and other targeting moieties, such as peptides possessing an Arginine-Glycine-Aspartic acid (RGD) amino acid sequence or a Tyrosine-Isoleucine-Serine-Arginine-Glycine (YISRG) motif; growth factors including epidermal growth factor, vascular endothelial growth factor and fibroblast growth factor; viral surface ligands such as sialic acid and N-acetylneuraminic acid derivatives; cell receptor ligands such as folate, methotrexate, pteroic acid, estradiol, estratriol, testosternone, and other hormones; mannose-6-phosphate, sugars, vitamins, tryptophan. Antibodies are preferably monoclonal antibodies directed at cell-specific surface antigens; suitable targeting moieties include not only complete antibodies but also antibody fragments containing the active antigen-binding sequences, such as Fab'2 fragments, Fab' fragments, or short chain peptide analogues of the active antigen binding sequences of such antibodies.

Examples of virus-targeting moieties include small molecule ligands that bind to a virus, such as aminoalkyladamantanes, Fuzeon™, PRO-542, BMS-488043, sialic acid, 2-deoxy-2,3-didehydro-N-acetylneuraminic acid, 4-guanidino-Neu5Ac2en (zanamivir), oseltamivir, RWJ-270201, oligopeptides, oligosaccharides, and glycopeptides that bind to viral surfaces, and antibodies and antibody fragments directed at virus-specific surface antigens. In preferred embodiments, the present invention provides π-polymers bearing ligands for viral neuraminidase or hemagglutinin. It is well-established that such polymers have antiviral properties in their own right; see for example T. Masuda et al., Chemical & Pharmaceutical Bulletin 51:1386-98 (2003); M. Itoh et al., Virology 212:340-7 (1995), and Reece et al., U.S. Patent No. 6,680,054 (2004). The hydrophobic cores of the antiviral polymers and polymer aggregates of the present invention may optionally be loaded with one or more conventional antiviral drugs, which are advantageously released in the vicinity of the viral particle.

Other attachment groups of medical relevance may be small chemicals, peptides, antibodies or antibody fragments, enzymes, or active pharmaceutical ingredients, that may affect biological processes such as hormones or hormone agonists or antagonists, substances that interfere with virus binding, substances that interfere with cell cycle or cellular processes after intracellular entry. Cells of unicellular and multicellular organisms, including bacteria, fungi, higher animals, and plants, may be targeted. Biotin may be attached to the π-polymer and used as an attachment point for avidin- and streptavidin-coupled proteins, peptides, and other targeting or pharmacologically active agents, such as antibodies, growth hormones, imaging reagents.

"Matrix" refers to organic or inorganic materials, surfaces, and deposits, such as glass, silica or metal surfaces, extracellular matrix, protein deposits such as amyloid plaques of various kinds, cell surface, virus surface, and general homogeneous or heterogeneous surfaces that may or may not be well characterized, including prions.

Examples of glass or silica matrix attachment moieties include various halosilanes, alkoxysilanes, acylsilanes, as well as chemicals exhibiting such functional groups including polymers. Other attachment groups can be devised based on the particular physico-chemical characteristics of the matrix. Suitable attachment moieties, for example those used in the coating of stents, are known to those skilled in the art.

In a third aspect of the invention, the branch point moieties B are connected to other branch point moieties elsewhere in the polymer chain, so as to form a crosslinked hydrogel structure. Such crosslinking may be effected by reacting the polymer with multifunctional moieties that contain homofunctional or heterofunctional groups, at least one of which reacts with X or a reactive group on C located on a first branch point moiety, and at least one of which reacts with X or with a reactive functional group present on C at a second branch point moiety. Cross-linking may also be made via a link to the terminal functional groups of the polymer chain A. Such crosslinked polymers may optionally contain reactive functional groups suitable for attachment of drug molecules or targeting moieties.

The branch-point moieties are the conjugates of dithiothreitol (DTT), dithioerythritol (DTE), or 2,3-diaminobutane-1,4-dithiol with two molecules of maleic acid. The combination of this branch-point moiety with polyethylene glycol as the moiety A generates the polymer backbone of Formulas **3** and **3a** 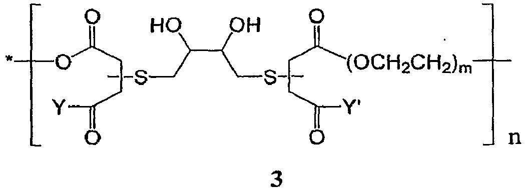 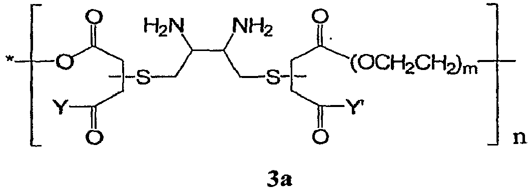 wherein Y and Y' may be the same or different, and are preferably selected from OH, NH₂, ONH₂, NHOH, and NHNH₂. In a preferred embodiment, the hydroxyl or amino groups of the dithiol are the reactive groups X, serving as attachment points for targeting or drug moieties, while the functional groups Y and Y' serve as attachment points for C moieties. Alternatively, the groups Y and Y' may serve as attachment points, while the hydroxyl or amino groups are used to attach the C moieties.

Formulas **3** and **3a** are intended to convey that each sulfur atom may independently be attached alpha or beta to a PEG ester carbonyl group. The invention encompasses single isomer compositions as well as mixtures of regioisomers at one or both C-S bonds. Furthermore, due to the four asymmetric carbons in Formula 1, the invention encompasses all chiral, meso, and diastereomeric isomers and mixtures thereof.

Also disclosed herein is the Diels-Alder adduct of acetylene dicarboxylic acid and a furan serving as branch point moiety. For example, the polyester **4** derived from PEG and acetylenedicarboxylic acid is known to undergo Diels-Alder reactions with furans (M. Delerba et al., Macromol. Rapid Commun. 18(8):723-728 (1997)). Thus, it may be subjected to a Diels-Alder reaction with a 3,4-disubstituted furan to generate a species such as **5**, and polymer **5** can be modified by hydroxylation or epoxidation to provide reactive groups (e.g., X and X' in Scheme 1).

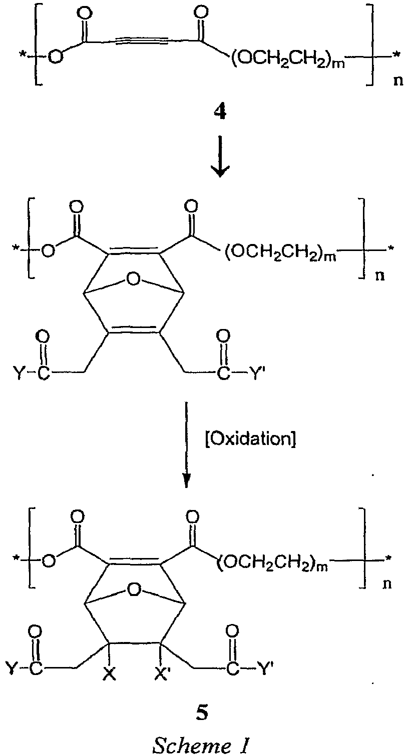

Similarly, reaction of PEG with ethylenediamine tetraacetic acid dianhydride will provide a polyester of formula **6**:

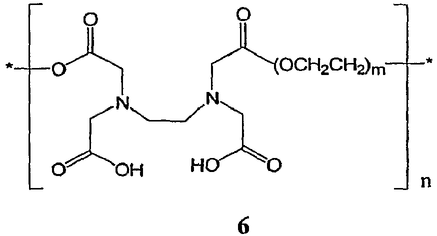

Other branch point moieties disclosed herein are derived from tartaric acid, acetylenedicarboxylic acid, nitrilotriacetic acid, 3,4,3',4'-diphenyl sulfone tetracarboxylic acid dianhydride, 3,4,3',4'-diphenyl ether tetracarboxylic acid dianhydride, pyromellitic dianhydride, alkanedithiols such as 1,2-ethanedithiol and 1,4-butanedithiol, bis(2-mercaptoethyl)ether, 2-mercaptoethylsulfide, dimercaptopropanol, dimercaptopurine, dimercaptothiadiazole, dimercaptosuccinic acid, benzenedimethanethiols, benzenedithiols, dihalogenated benzenedimethanethiols, dihalogenated 4,4'-thiobisbenzenethiol.

Where Y and Y' are OH, hydrophobic groups C may be linked to the polymer by amidation or esterification of the carboxylic acid groups. The hydrophobic groups C are preferably relatively small (C₈-C₂₀) and predominantly hydrocarbon moieties, and may be linear or branched or contain one or more rings. Examples include covalently attached moieties derived from the C-H molecules n-octanol, n-decanol, n-dodecylamine, n-pentadecylamine, cholesterol, and cholic acid. Although the polymers of the invention are represented, for convenience, as having at most two different hydrophobic side chains, is should be understood that mixtures of two or more hydrophobic compounds may be used to introduce a variety of hydrophobic side chains into a particular polymer.

As one specific example, a polymer of formula **2**, where X = OH and r = 2, was prepared by reacting a polyethylene glycol with maleic anhydride to form the polyester **7,** followed by reaction with dithiothreitol to form **8**. The acid **7** was then amidated with n-octadecylamine to form the desired comb polymer **9** (Scheme 2). The DTT-derived amide comb polymers represented by formula **9** are referred to herein as "π-Polymer A"; the specific polymer **9** in Scheme 2 is designated "C₁₈-π-Polymer A".

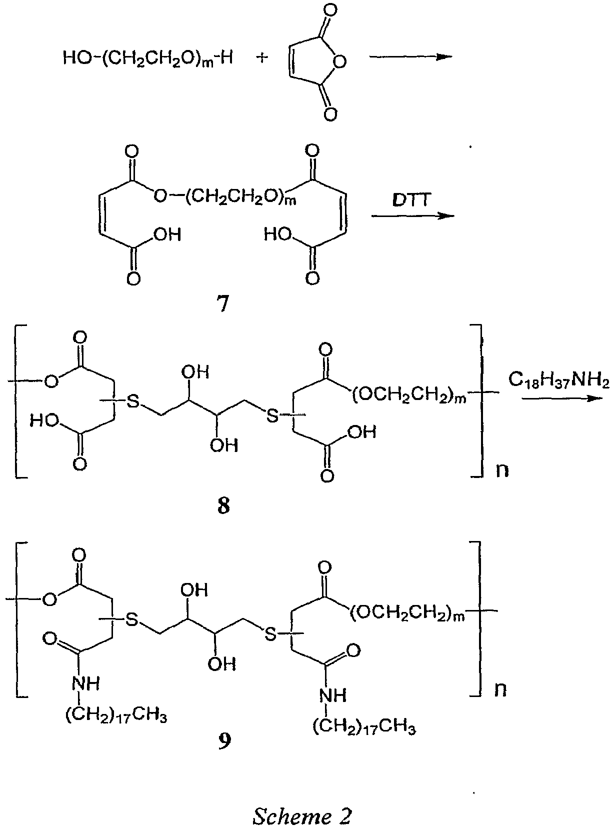

Substitution of 2,3-bis(t-butoxycarbonylamino)butane-1,4-dithiol (prepared by the method of DuPriest et al., U.S. Patent No. 4,755,528) for dithiothreitol leads, after deprotection, to the corresponding amino-functionalized π-polymer **9b** (Scheme 3).

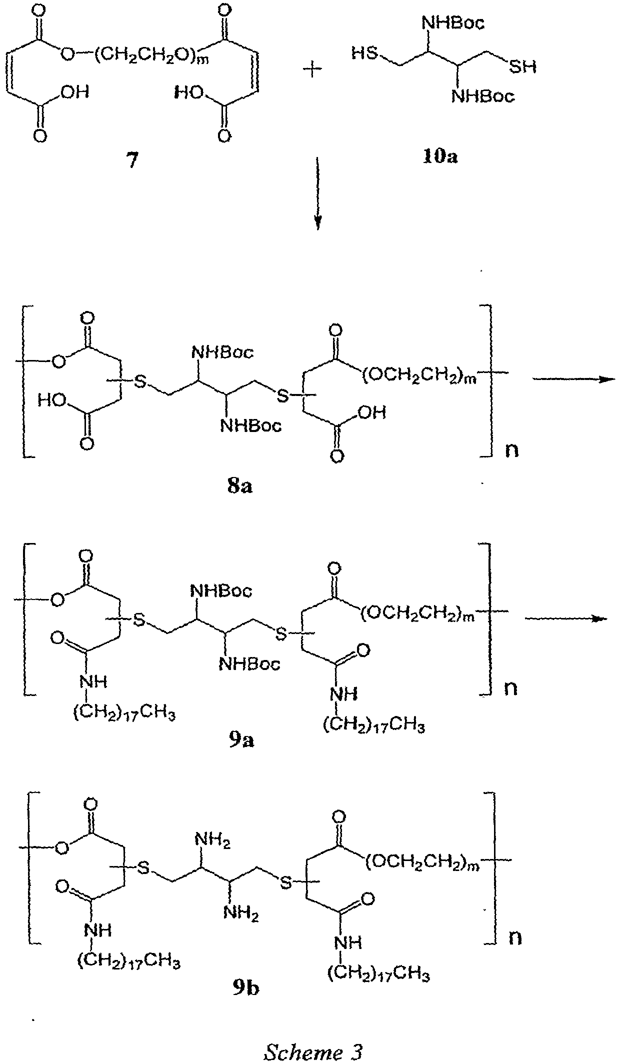

Use of the butanedithiol **10c** likewise leads the polymers of general structure **9c,** with spacer groups L in place for subsequent attachment of targeting moieties (Scheme 4). The spacer groups L may be any of the spacer groups known in the art for use in attaching ligands or labels to substrate molecules, including C₂ to C₂₀ alkylene and oligo(ethylene glycol) spacers having one to ten -CH₂CH₂O- units.

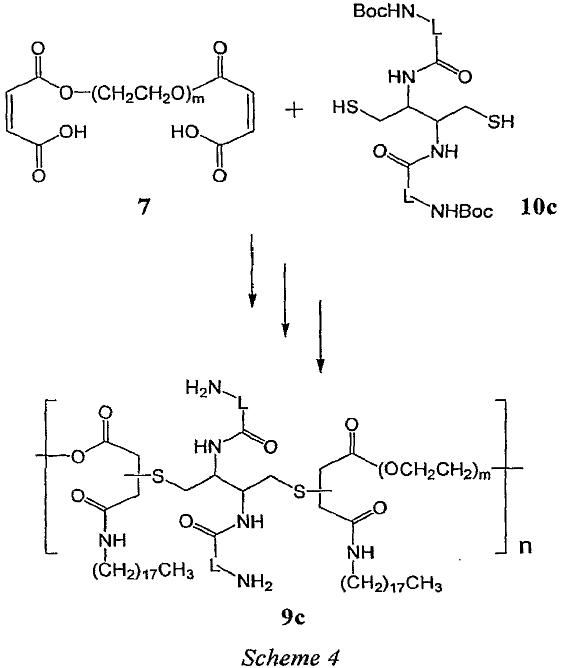

In other embodiments, a PEG polymer with terminal amino groups may be used to prepare examples having amide bonds between the A and B units, as shown in structures **10-14** below. Each of these polyamides may be derived via reaction of the PEG diamine H2N-(CH₂CH₂O)ₘCH₂CH₂-NH₂ with the appropriate cyclic anhydride:

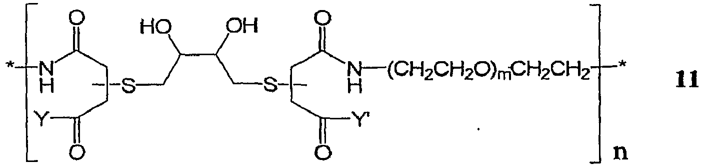

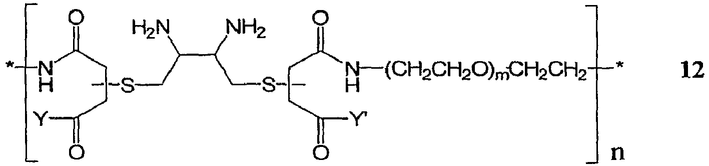

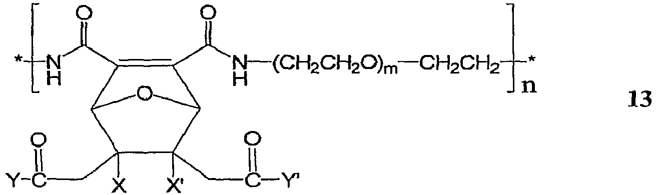

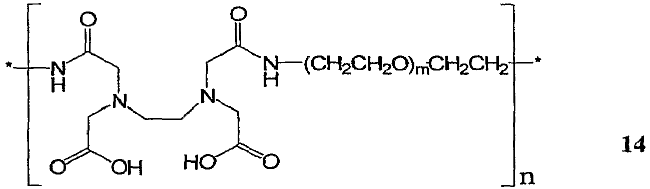

Under mild conditions, the above amido acids are the expected products. Upon heating, imide formation can be expected, leading to polymers with fewer reactive groups but still suitable for attachment of hydrophobic C moieties. Alternatively, the pendant side chains C can be added to the ends of the polymer A blocks, and the branch point moieties can come into existence at the time of polymerization (Scheme 5).

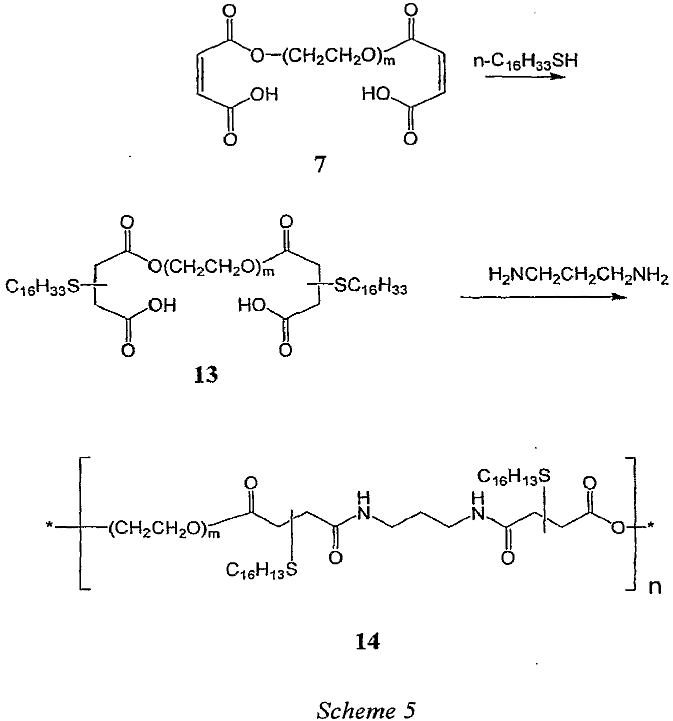

In addition to simple diamines such as 1,3-diaminopropane, as shown in Scheme 5, diamines having (optionally masked) reactive functional groups X may be employed, leading to polymers **15** suitable for attachment of targeting moieties (Scheme 6). In the formulae below, p may range from 0-4, and each X is independently the same or different from any other group X that may be present. A reactive group X need not be pendant, but may for example be an NH group within the chain of atoms that makes up the diamine, as in the monomer H₂N-(CH₂)₃-NH-(CH₂)₃-NH₂.

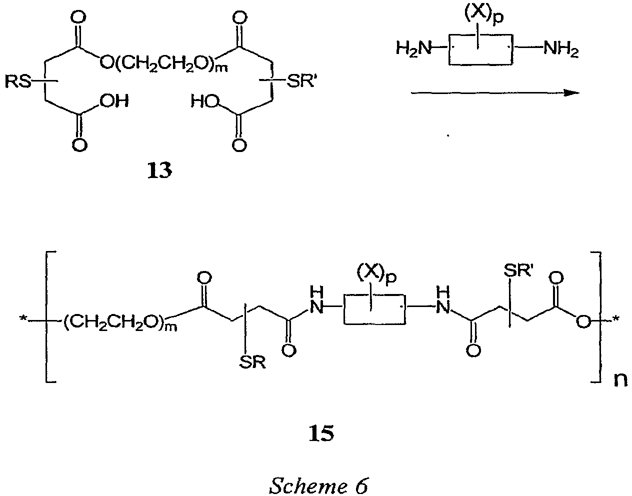

Certain of the π-polymers prepared as above possess reactive groups X suitable for further derivatization, to attach targeting moieties such as small molecules, peptides, nucleotides, sugars, antibodies, etc., or to effect crosslinking of the polymer chains via bifunctional or multifunctional crosslinking agents. In particular embodiments, partial derivatization of the reactive groups on the polymer chain is carried out to generate π-polymers having a variety of different reactive groups, which permits attachment of a variety of targeting and drug moieties to a single polymer chain. Thus, addition of a sub-stoichiometric amount of acryloyl chloride (or maleic anhydride) to the π-polymer of Example 1 will provide a polymer with both acryloyl (or maleyl) groups and residual hydroxyl groups. Subsequent Michael addition of a sub-stoichiometric amount of a mercapto-carboxylic acid, for example HS-(CH₂)₃-COOH, would provide a polymer with hydroxyl, acryloyl, and carboxyl groups. Addition of cysteine introduces amino and carboxyl groups, in addition to any residual reactive groups left behind by sub-stoichiometric amounts of reagents.

Another approach to poly-functional π-polymers involves the deliberate omission of a fraction of the hydrophobic chains C. The π-polymer of Example 1, for example, can be prepared with unreacted carboxylic acid groups by the simple expedient of limiting the amount of pendant-forming alkylamine in the amidation step. Yet another approach is amidation with a mixture of amines, a fraction of which contains a reactive group X. Also, under appropriate conditions (excess maleic anhydride in Step A and excess DTT in Step B), a polymer preparation having a desired population of free thiol groups may be generated.

The π-polymer of Example 1 contains, by design, hydroxyl groups derived from the DTT moiety in the backbone, which serve as reactive groups X. Esterification of these groups with acryloyl chloride or methacryloyl chloride in aqueous media in the presence of a carbonate/bicarbonate buffer results in acryloyl substitution on the -OH groups. The acrylated polymer can be readily subjected to radical polymerization (with or without added radical monomer such as an acrylic compound or crosslinker such as a bisacrylic compound) to obtain hydrogels suitable for controlled drug delivery (acting as polymer depots or reservoirs) and for topical applications (such as skin patches or ointments). The acryl group can also be subjected to a Michael addition, in particular, with a thiol, such as that of a cysteine residue in a protein, enzyme, peptide, antibody, Fab'2 fragment or Fab' fragment, or other targeting moiety (Scheme 7).

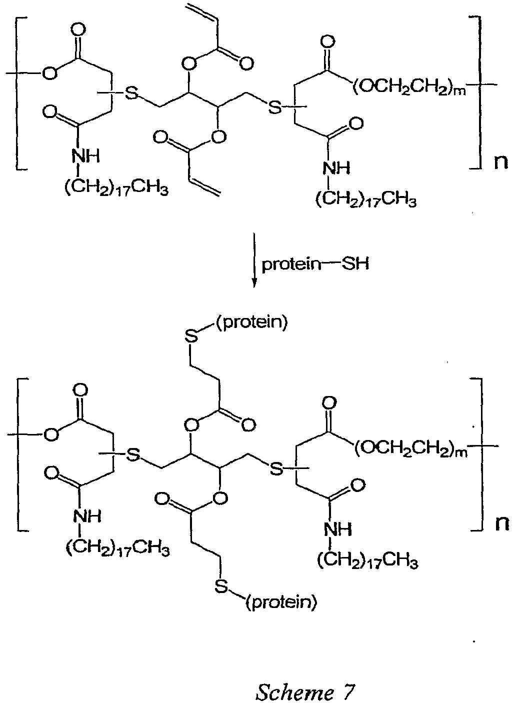

A π-polymer possessing reactive hydroxyl groups, after drying, can also be esterified with maleic anhydride to attach the maleate group, a Michael acceptor, simultaneously generating a free carboxylic group. In the resulting polymer, the maleic double bond is available for a Michael addition, in particular, with a thiol, such as that of a cysteine residue in a protein, enzyme, peptide, antibody, Fab'2 fragment or Fab' fragment, or other targeting moiety. (Scheme 8), and the carboxyl group is available for coupling to amino groups of drugs or ligands, or the lysine residues in proteins and peptides.

A different moiety may further be attached to the newly introduced (or previously available) carboxylic group via amidation. Thus at least two different targeting moieties can be attached even under saturating reaction conditions (i.e. the moiety to be attached is present in stoichiometric excess).

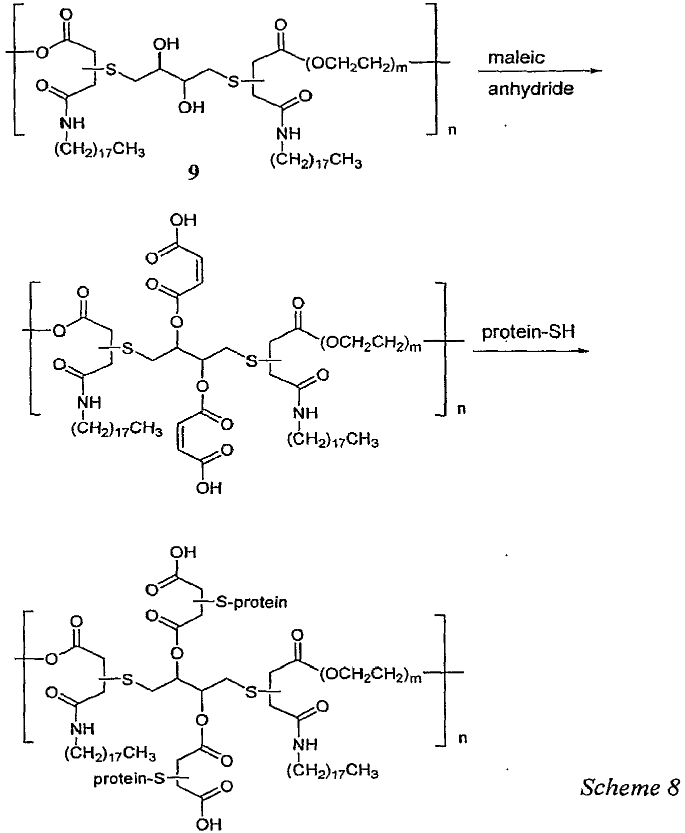

Polymers bearing pendant carboxylate groups may be amidated with amines under typical coupling conditions, and they may also be converted to isocyanate groups via the Curtius rearrangement and then coupled with amines or alcohols to form ureas and carbamates, respectively. Such reactions may be used to introduce the hydrophobic groups C, or to attach targeting moieties.

Free amines can be introduced in the polymer by at least partially reacting one of the reactive groups with a diamine. The diamine must be chosen so that one of the amine groups is either protected or unreactive under the conditions of the reaction. The latter can frequently be accomplished by using ethylenediamine at a pH of about 7.5, since the pKa's of the two amino groups differ considerably. Preferably, this amidation is carried out as a separate step after the introduction of the hydrophobic pendant groups. A peptide or another molecule having a carboxylic group can then be attached by amidation at this free amine.

Thus, even under saturating conditions, as many as three different peptides or other targeting moieties can be attached to the π-polymer: one via the thiol, one via the amine or hydroxyl, and one via the carboxylic acid group.

Hydroxyl and thiol groups can also be converted to primary amines by reaction with aziridine or a haloalkyl amine (such as bromoethylamine or chloroethylamine). Amidation with cysteamine will introduce a disulfide, which can be directly reacted with by the cysteine of a peptide or antibody to attach the peptide or antibody; or can be first reduced, e.g., with aminoethanethiol or DTT, for further reaction with a peptide or antibody.

By performing partial reactions, one can introduce additional reactive functional groups to a polymer of the invention, including but not limited to (1) thiol-reactive groups such as acrylic or maleic acid derivatives, (2) carboxylic-acid reactive groups such as amino or hydroxyl, (3) amine-reactive groups such as carboxyl, and (4) disulfide-reactive groups such as mercapto. The number of such added functional groups per polymer molecule may range from 1/r up to several multiples of r, depending on the reagent used and the quantity used.

Alternatively, two or more specific ligands can be attached to improve specificity of binding to say, a virus, or cell surface. Two or more specific ligands can also be used so as to cause an interaction between different cellular targets, for example, one ligand may target a virus particle, and another ligand may facilitate binding to a phagocyte, thereby bringing virus particle into proximity or contact with the phagocyte and promoting phagocytosis.

Such derivatization allows the attachment of three or more distinct targeting and/or therapeutic moieties to the polymer, through different functional group attachments (such as amine, carboxylate, and thiol). Thus, one may attach a tissue-specific targeting agent, an imaging agent, and a therapeutic agent to a single polymer chain, and subsequent self-assembly of the polymer will yield a targeted therapeutic whose distribution and efficiency of targeting can be monitored.

Attachment of ligands to the repeating units of the polymers of the invention affords multivalent display of the ligand on the polymer chain and on the nanoparticles surface. Multivalent display often leads to great increases in affinity for the target. For example, multivalent antibodies can be far more effective in clearance of their targets than the normal divalent antibodies. Carbohydrate-binding proteins and carbohydrates are known to be multivalent in nature, and ineffective if monovalent. Similarly, multivalent peptide and carbohydrate targeting moieties will be far more effective than the monomer alone. The increase in MW due to attachment to the polymer results in reduced renal clearance rates of peptides and other ligands. In addition, the PEG backbone affords to the peptide benefits similar to those of PEGylation, including evasion of immune surveillance.

Further, a multivalent targeting moiety will decorate a multivalent target (say, a virus particle) and neutralize it far more effectively than the monomeric targeting moiety. The ability to display multiple (different) peptides in multivalent format will lead to enhanced specificity. For example, a truly HIV-specific (HIV virus-binding) polymer can be built by attaching a peptide corresponding to the CD4 binding region, and another peptide corresponding to the CCR-5 or CXCR-4 binding region of the virus, and possibly a third peptide corresponding to the other receptor (CXCR-4 or CCR-5 respectively). Such a polymer could completely mask the virus's binding regions and render the virus unable to attach to cells and thereby non-infective. In addition, the surfactant properties of the polymer would lead to destabilization of the virus structure itself upon binding. Instead of peptides, small molecules that interfere with the same binding patterns (CD4, CCR-5, CXCR-4) or a mixture of peptides and small molecules, preferably with complementary activities, can be employed. The resulting polymers will render any free virus ineffective, and thus may be ideal for stopping spread of infection, by using them as components of condom lubricants and the like. In addition, such polymers may be injected into patients to reduce the HIV burden.

Generally, when a polyfunctional reagent such as DTT is employed, there may be partial cross-linking of polymer chains via esterification of the carboxylic acid with DTT or similar side reactions. Secondary hydroxyl groups in the central region of the PEG chains, e.g. those associated with bisphenol A diglycidyl ether residues, may also contribute to cross-linking if they are present in the PEG starting material. The resulting crosslinked hydrogel structures are also useful materials. For example, by suitably increasing the extent of this crosslinking or by explicit cross-linking using alternative crosslinkers (such as bisoxiranes, for example), materials can be made that are flexible hydrogels which can serve as repository depots for drugs. By suitably modifying the materials (e.g. lower PEG length, greater open carboxylic groups, and incorporation of suitable acrylic groups) either linear or crosslinked hydrogel materials can be made that can serve as repositories that can be supported either immobilized on devices such as stents or absorbed in devices such as pads for adhesive patches or subdermal insertion patches. In general, such crosslinked materials will be suitable for controlled release rather than enhanced, targeted release.

The comb polymers of the invention are useful for solubilizing, in aqueous solvent systems, sparingly water-soluble materials. The method of solubilizing a substance in an aqueous solvent comprises contacting the sparingly-soluble substance with a comb-type polymer of the invention in the presence of water, so as to form a water-soluble complex of the substance and the polymer. Alternatively, the polymer and the substance to be solubilized may be combined in a two-phase aqueous-organic emulsion, and the organic solvent removed by evaporation. An exemplary process is described in U.S. Patent No. 6,838,089. It is believed that in most cases, the polymer self-assembles into nanoparticles having the sparingly-soluble substance dissolved among the hydrophobic C chains that coalesce at the core of the particles, while the A blocks form a hydrophilic corona that sufficiently lowers the interfacial free energy to permit an aqueous suspension of the particles to remain stable.

In some cases, the sparingly-soluble substance may not entirely dissolve in the core, but may exist as a solid nanoparticle surrounded by and suspended in the C chains at the core of the particles. For the purposes of the present invention, this is a differences of degree, as the practice of the invention does not rely on any particular degree of mixing of the C chains with the sparingly-soluble substance. The substance may in some cases dissolve at the molecular level among the C chains, but in other cases it may exhibit any degree of phase separation from the C-chain environment. In some cases, it can be expected that the system will move from one state to the other as a function of temperature.

The solvating power of the hydrophobic core of the polymer particles can be modified by modifying the hydrophobic C moieties. Suitable modifications include the introduction of one or more hydrophilic substituents, such as hydroxyl, ether, amide, and cyano functional groups, in order to increase the polarity and/or polarizability of the hydrophobic core.

Sparingly-soluble materials that can be rendered soluble by these polymers include fat-soluble vitamins and nutrients, including vitamins A, D, E and K, carotenes, cholecalciferol, and coenzyme Q; insoluble drugs such as docetaxel, amphotericin B, nystatin, paclitaxel, doxorubicin, epirubicin, rubitecan, teniposide, etoposide, daunomycin, methotrexate, mitomycin C, cyclosporine, irinotecan metabolite (SN-38), statins, and steroids; dyes, photodynamic agents, and imaging agents, and nucleic acids, nucleic acid analogues, and nucleic acid complexes. Nucleic acid analogues include species such as thiophosphates and peptide nucleic acids; nucleic acid complexes are ionic complexes of oligonucleic acids with a substantially charge-neutralizing amount of cationic or polycationic species.

For the purposes of this disclosure, a drug that is insoluble at neutral pH is considered "sparingly soluble", because there is in many cases a need for a neutral pharmaceutical composition. For example, ciprofloxacin is reasonably soluble in water at a pH below 4.5, but this pH can be highly irritating when the drug is formulated for ocular administration. A polymer of the present invention will solubilize ciprofloxacin in normal saline at pH 7. Also, for the purposes of this disclosure, "sparingly soluble" should be understood to refer to any substance whose solubility in an aqueous vehicle is such that an increase in solubility would yield an improved or more-useful composition. Thus, a drug that is moderately soluble, e.g. to the extent of 2g/liter, is "sparingly soluble" if a unit dose for intravenous administration is

As a result of the ability of the polymers of the invention to solubilize pharmacologically active species, the present invention also provides pharmaceutical compositions, which comprise one or more π-polymers of the invention in combination with a therapeutically effective amount of one or more pharmacologically active agents. The polymers of the invention can render effective what would otherwise be an ineffective amount of a pharmacologically active agent. For purposes of this disclosure, therefore, a "therapeutically effective amount" is the amount of agent that renders the overall composition effective.

### EXPERIMENTAL

### 1. General procedures.

The invention also provides processes for the preparation of the comb polymers of the invention. Synthesis of these polymers is readily carried out by one skilled in the art of organic synthesis, by following the procedures described below. The key starting material is polyethylene glycol, which is preferably dried before use. This is conveniently done by stirring molten PEG under vacuum at an elevated temperature, until bubbles stop forming. This may take 8-12 hours, depending on the quality of the PEG. Once dried, the PEG can be stored under argon indefinitely. Commercially available industrial and research grades of PEG may be employed in making the polymers of the invention, for example the polydisperse "PEG 1500" of commerce having a molecular weight distribution of 1430 - 1570. Such material may incorporate bisphenol A diglycidyl ether, which introduces secondary hydroxyl groups at the center of the PEG chain. In order to ensure that the polymers of the invention have the most reproducible and consistent properties, the PEG is preferably free of bisphenol A, and of low dispersity. Most preferable are PEG polymers that are >95% monodisperse, such as are commercially available from Nektar Therapeutics (formerly Shearwater Polymers), Huntsville AL, and Polypure AS, Oslo, Norway. An example of a particularly preferred PEG is "PEG-28" from Polypure, which is >95% HO(CH₂CH₂O)₂₈H, molecular weight 1252.

All reactions are carried out under an inert atmosphere such as nitrogen or argon, with magnetic or preferably mechanical stirring.

In step A, dry PEG is melted, and maleic anhydride (2 moles per mole of PEG) is added with stirring. The quantity of maleic anhydride should match the number of PEG terminal hydroxyl groups as closely as possible. A shortage of maleic anhydride will result in hydroxyl-terminated polymer chains, whereas an excess of maleic anhydride will consume thiol groups in the next step, leading to premature chain termination and terminal carboxyl groups. The reaction temperature is not critical, and the process can conveniently be carried out at temperatures between 45°C and 100°C. The preferred temperature of the reaction is between 65°C and 90°C. If elevated temperatures are employed, the maleic anhydride tends to sublime, and steps should be taken to see to it that the maleic anhydride remains in solution. Minimizing headspace and submerging the reaction vessel in an oil bath are effective methods.

Depending on the temperature selected, the reaction may be completed in 2 hours or less or can be conducted overnight. The reaction may be monitored by TLC on silica gel plates, and is continued until after the disappearance of the maleic anhydride. Visual contrast, UV, and iodine staining can all be used to examine the TLC plates.

In step B, the crude PEG bis-maleate ester produced in step A is combined with dithiothreitol (DTT) and N,N,N',N'-tetramethylethylenediamine (TEMED) (with added water, if necessary for fluidity), and the mixture stirred at 70°C. The reaction is complete within 30 min, as indicated by the rapid increase in viscosity. The molecular weight of the product will be reduced if more or less than the optimal amount of DTT is employed. The molecular weight of the product can also be reduced, if desired, by replacing TEMED with a less effective tertiary amine base such as TEA.

In step C, sufficient water is added to the reaction mixture to reduce viscosity, and 0.1 mol N-hydroxysuccinimide (NHS) and 1.05 mol hexadecylamine per mol carboxylic acid groups in the polymer are added. (This amount of NHS appears to optimally minimize the extent of side-reactions.) An excess of N-(3-dimethylaminopropyl)-N'-ethylcarbodiimide (EDC) (1.4mol EDC per mol of carboxylic acid groups) is then added in portions, with additional water as added as necessary to maintain stirring. The pH of the reaction mixture is maintained above 7, and preferably between 9- and 11, to optimize the reactivity of the alkylamine. With dodecylamine, this reaction can be conducted at about 40~45°C, whereas with octadecylamine, the temperature is ca. 55°C-57°C. The reaction is followed by TLC until a constant level of left-over alkylamine is observed, typically after running overnight.

The reaction mixture is acidified to a pH from about 3.0 to about 4.5 and stirred at room temperature for about 24 hours to destroy unreacted EDC, then titrated to a pH of 7.0 using 1N NaOH. The final reaction mixture is centrifuged at about 800 xg for 1 to 3 hours, to remove solid contaminants and by-products.

After centrifugation, the supernatant can be chromatographed on a GPC column (Toyopearl™, Sephadex™, Sephacryl™, Biogel™, and the like). The π polymers are amphipathic materials, however, and will exhibit affinity for most GPC column packings, which complicates the removal of contaminants. Alternatively, the polymer may be chromatographed on a large-pore hydrophobic interaction column (e.g., TOYOPEARL™ Phenyl 650C, Toshoh Biosciences, Montgomeryville, PA, U.S.A.), eluting with a gradient of methanol in water. Preferably, the reaction mixture is dialyzed against several changes of acidified and neutral water to remove low-molecular-weight starting materials and reaction by-products.

The reaction mixture may also be extracted with butanone, isopropanol, butanol or other polar organic solvents to remove organic impurities, but substantial amounts of the amphiphilic polymer are lost to the extraction solvent. Preferably, the reaction mixture is subjected to ultrafiltration using suitable membranes to fractionate the product into molecular weight grades, such as 5kDa to 10kDa; 10kDa to 30kDa, 30kDa to 50kDa, etc. depending upon the cutoff of the filtration membrane employed. An aqueous solution of the polymer may be subjected to dead-end filtration so as to produce a sterile or virus-free solution, depending upon the choice of filtration membrane or media.

### 2. Synthesis of π-polymers

### Example 1: PEG-Di(alkylamidosuccinyl)dithioether Medium Molecular Weight Polymer (C16-π-Polymer A)

Polyethylene glycol (PEG-1500, Sigma Chemical Co.) was dried under vacuum at 80°C until bubbles stopped forming. (8-12 hours, depending on the quality of the PEG.) The dried PEG can be stored desiccated under argon indefinitely.

The dried PEG was melted under argon on an oil bath, and maleic anhydride (2 moles per mole of PEG, corrected for impurities) was added gradually with stirring. The mixture was stirred under argon at 90°C. Because maleic anhydride tends to sublime, the head space was minimized and the entire reaction vessel was kept at the reaction temperature. Any condensed maleic anhydride on the vessel walls was scraped back into the reaction mixture. The progress of the reaction was monitored by TLC on silica gel plates, using ethanol and hexane as solvents separately, with UV visualization and iodine staining. The reaction was continued for one hour past the disappearance of the maleic anhydride.

The crude PEG dimaleate was diluted with two volumes of water. A solution of dithiothreitol (DTT, 1.01 equivalents per equivalent of PEG) and N,N,N',N'-tetramethyl-ethylenediamine (TEMED, 1.02 equivalents) in water (2 volumes water per volume of TEMED) was then added to the reaction mixture with stirring. The reaction was stirred at 70°C under argon for 2.5 hrs, left at room temperature overnight, and then stirred again at 70°C for 2 hours. The reaction was monitored by TLC and was judged complete upon complete disappearance of the DTT.

Water was added to the above reaction mixture to reduce the viscosity, until the mixture could be stirred (at ca. 25% solids), the mixture was stirred at 65°C under argon, and N-hydroxysuccinimide (0.1 mol per mol carboxylic acid groups in the PEG-dimaleate-DTT polymer) was added, followed by hexadecylamine (1.05 mol per mol carboxylic acid groups in the polymer) and N-(3-dimethylaminopropyl)-N'-ethylcarbodiimide (EDC, 0.56 mol per mol carboxylic acid groups in the polymer). The mixture was stirred under argon for 1 hour and a second portion of EDC (0.56 mol per mol carboxylic acid groups in the polymer) was added. After another hour, a third portion of EDC (0.28 mol per mol carboxylic acid groups in the polymer, for a total of 1.4 mol EDC per mol of carboxylic acid) was further added to account for loss of EDC to hydrolysis. Additional water was added as necessary to maintain fluidity, as the added solids made the suspension difficult to stir, and the pH was maintained between 8 and 10 by addition of 1N NaOH as needed. The mixture was stirred at 65°C under argon overnight, monitored by TLC (silica with ethanol) until the alkylamine appeared to have reached a steady concentration, and was then stirred for an additional 4 h. The reaction mixture was then acidified with 1N HCl to a pH of about 4.5, stirred for 24 h to destroy unreacted EDC, and adjusted to pH 7.0 by dropwise addition of 1N NaOH. With dodecylamine, this reaction was conducted at about 40~45C, whereas with octadecylamine, the temperature was preferably 55 - 57°C.

The mixture was transferred to centrifuge bottles and spun in a benchtop centrifuge at about 800xg for 2 hours to separate residual solids. After centrifugation, the reaction mixture was extracted with isopropanol to remove organic impurities. Ultrafiltration is preferred as an alternative to isopropanol extraction.

By this method, the following amino compounds are conjugated to the polymer:
Example 1a: undecylamine
Example 1b: octadecylamine
Example 1c: 4-nonylbenzylamine
Example 1d: 3-[(4-phenoxy)phenyl]propylamine

### Example 2: PEG-Di(alkylamidosuccinyl)dithioether High Molecular Weight Polymer

The procedure outlined in Example 1 was followed, except that 0.55 mol DTT and 0.55 mol TEMED per mol maleic anhydride were used. Vigorous stirring was necessary as the viscosity built up rapidly. It appeared that most of the reaction was complete within 5-10 minutes, followed by slow completion over the next 4 hours as the temperature was raised from 55°C to 80°C.

### Example 3: PEG-Di(alkylamidosuccinyl)dithioether Polymer

The procedure outlined in Example 1 was followed, except that 1.5 mol dodecylamine per mol of carboxylic acid groups in the polymer was employed. N-hydroxysuccinimide (NHS, 1.0 mol per mol of carboxylic acid groups) and 1,1'-Carbonyldiimidazole (CDI, 3.0 mol per mol of carboxylic acid groups) were added, and the reaction was stirred at 80°C for 4 hours and worked up as above.

By this method, the following amino compounds are conjugated to the polymer:
Example 3a: undecylamine
Example 3b: tetradecylamine
Example 3c: octadecylamine
Example 3d: dehydroabietylamine
Example 3e: cholesterol 2-aminoethyl ether
Example 3f: 10-phenoxydecylamine
Example 3g: sebacic acid hydrazide
Example 3h: oleic acid hydrazide
Example 3i: dehydroabietic acid hydrazide
Example 3j: cholic acid hydrazide
Example 3k: palmitic acid hydrazide

### Example 4: PEG-co-(alkylamidosuccinate) Polymer

A solution of PEG (6.66 mmol) and triethylamine (2.32 ml, 16.65 mmol) in dry diethyl ether (10 ml) is cooled at 0 °C under argon and treated dropwise with methanesulfonyl chloride (1.03 ml, 13.32 mmol). Stirring is continued for 1 h at 0 °C and then at room temperature for 2 h. The ether is evaporated and dry acetone (15 ml) is added to the residue in order to precipitate the triethylamine hydrochloride, which is filtered from the solution. The filtrate is treated with lithium bromide (2.31 g, 26.64 mmol) and heated to reflux for 20 h. Then the mixture is diluted with hexane and filtered through a short column of silica (3 cm) covered with Celite™ (0.5 cm), and eluted with hexane. The filtrate is dried, filtered and evaporated to leave α,ω-dibromo-PEG an oil.

α,ω-Dibromo-PEG is reacted with one equivalent of 2,2-dibutyl-4,5-bis(methoxycarbonyl)-1,3,2-dioxastannolane by the method of Godjoian et al., Tetrahedron Letters, 37:433-6 (1996). The resulting dimethyltartrate-PEG polyether is saponified with KOH in methanol, and then amidated with dodecylamine or hexadecylamine as in examples 1 and 3 above, or with the amines in examples 3a-3k.

### Example 5: PEG Copolymerization with EDTA dianhydride

Dry PEG is reacted with ethylenediaminetetracetic acid dianhydride by the method described in Example 1, and is then amidated with dodecylamine as in Example 1 or hexadecylamine as in example 3, or with the amines in examples 3a-3k.

In the same manner, the following dianhydrides are co-polymerized with PEG and subsequently amidated:
Example 5a: Naphthalenetetracarboxylicdianhydride
Example 5b: Perylenetetracarboxylicdianhydride
Example 5c: Benzophenonetetracarboxylicdianhydride
Example 5d: 4,4'-(Hexafluoroisopropylidene)diphthalic anhydride
Example 5e: Butane tetracarboxylic acid dianhydride
Example 5f: Bicyclo(2,2,2)oct-7-ene-2,3,5,6-tetracarboxylic dianhydride
Example 5g: Diethylenetetramine pentaacetic acid dianhydride
Example 5h: 3,4,3',4'-Diphenylsulfone tetracarboxylic acid dianhydride
Example 5i: 3,4,3',4'-Diphenyl ether tetracarboxylic acid dianhydride
Example 5j: Pyromellitic dianhydride

### Example 6A: PEG-diamine co-polymer with pendant thioethers.

PEG dimaleate, prepared as in Example 1, is reacted with dodecanethiol (two equivalents per equivalent of PEG dimaleate) using the same procedure as used for DTT in Example 1. No dilution is necessary, as no polymerization takes place, and the reaction is conducted in molten PEG-dimaleate. The TEMED catalyst is added and then the thiol is added. The reaction is followed by the disappearance of starting materials, using TLC. Temperatures up to the point where the loss of alkylthiol by vaporization becomes significant can be employed (up to ca. 100 °C). A slight excess of alkylthiol may be employed to fully saturate the maleic groups. The excess alkylthiol is driven off at the end of reaction by sparging with nitrogen or argon, and/or heating under vacuum, until none is detected by odor or by TLC.

By this method, the following thiols may be conjugated to PEG dimaleate:
Example 6Aa: mercaptosuccinic acid di-t-butyl ester
Example 6Ab: tetradecanethiol
Example 6Ac: hexadecanethiol
Example 6Ad: 2-mercaptoethanesulfonic acid
Example 6Ae: 3-mercaptopropanesulfonic acid
Example 6Af: 6-mercaptohexanoic acid t-butyl ester
Example 6Ag: 4-mercaptobenzoic acid t-butyl ester
Example 6Ah: mercaptoacetic acid t-butyl ester
Example 6Ai: 4-(t-butoxycarbonylamino)butanethiol
Example 6Aj: 3-(t-butoxycarbonylamino)benzyl mercaptan
Example 6Ak: 4-decylbenzyl mercaptan

Thiols having reactive functional groups are suitable for attachment of C chains, and/or the reactive functional groups may serve as attachment points (X) for targeting moieties.

### Example 6B: PEG-diamine co-polymer with pendant thioethers.

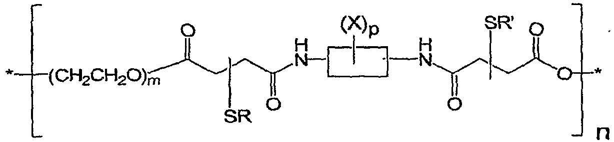

The thiol adduct obtained in Example 6A is amidated with 1,4-diaminobutane (one equivalent of diamine per two COOH groups), using the same procedure used for dodecylamine in Example 1, with dilution with water is as necessary to maintain the fluidity of the reaction mixture. Additional aliquots of EDC are added as necessary to ensure complete polymerization. By this method, the thiol adducts of Example 6A and 6Aa through 6Ak are converted to a PEG-diaminobutane polyamide.

By this method, the following diamines may be converted to a PEG polyamide (BOC = *t*-butoxycarbonyl):
Example 6Ba: 2-*(O*-BOC)-1,3-diamino-2-propanol
Example 6Bb: N',N"-di(BOC) hexaethylene tetraamine
Example 6Bc: N',N"-di(BOC) spermine
Example 6Bd: N'-BOC spermidine
Example 6Be: N',N",N"'-tri(BOC) pentaethylehe hexamine
Example 6Bf: agmatine
Example 6Bg: lysine t-butyl ester
Example 6Bh: 1,6-diaminohexane
Example 6Bi: 1,4-phenylenediamine
Example 6Bj: 1,3-phenylenediamine
Example 6Bk: 1,4-diaminobutane-2,3-diol acetonide

### Example 7: PEG-Di(alkylsuccinate)dithioether

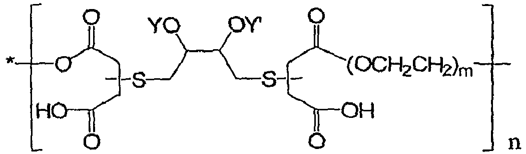

The 2,3-bis-O-hexadecyl ether of DTT (*meso*-2,3-bis(hexadecyloxy)butane-1,4-dithiol) is prepared by a modification of the procedure of S. Sasaki et al., Chem.Pharm.Bull. 33(10):4247-4266 (1985). This is added to PEG-dimaleate by the method of Example 1.

By this method, the following ether dithiols are coupled to the PEG polymer:
Example 7a: *meso*-2,3-bis(n-butoxy)butane-1,4-dithiol
Example 7b: *meso*-2,3-bis(4-nonylphenylmethoxy)butane-1,4-dithiol
Example 7c: *meso*-2,3-bis(biphenyl-4-methoxy)butane-1,4-dithiol
Example 7d: *4,6*-bis(decyloxy)benzene-1,3-dimethanethiol
Example 7e: *4,5*-bis(decyloxy)benzene-1,2-dimethanethiol
Example 7f: 3,4-bis(decyloxy)thiophene-2,5-dimethanethiol

### Example 8A: substituted PEG succinates

The method of Example 1 is followed, except that 2-dodecen-1-yl succinic anhydride is used in place of maleic anhydride. The dodecenyl substituent provides the pendant C chains in the final polymer.

By this method the following substituted succinic anhydrides are esterified with PEG:
Example 8Aa: isobutenylsuccinic anhydride
Example 8Ab: 2-octene-1-yl succinic anhydride
Example 8Ac: octadecenyl succinic anhydride
Example 8Ad: 3-oxabicyclo-hexane-2,4-dione
Example 8Ae: cyclohexanedicarboxylic anhydride
Example 8Af: phthalic anhydride
Example 8Ag: 4-decyl phthalic anhydride
Example 8Ah: hexahydromethylphthalic anhydride
Example 8Ai: tetrahydrophthalic anhydride
Example 8Aj: norbornenedicarboxylic anhydride
Example 8Ak: cantharidin
Example 8Al: bicyclooctenedicarboxylic anhydride
Example 8Am: exo-3,6-epoxy-1,2,3,6-tetrahydrophthalic anhydride
Example 8An: S-acetyl mercaptosuccinic anhydride

### Example 8B: PEG-Di(alkylamidosuccinyl)dithioether with pendant alkyl groups

By the method of example 1, the substituted PEG succinates obtained as described in Examples 8A and 8Aa through 8An are reacted with DTT.

By this method, the following dithiols are reacted with any of the substituted PEG succinates obtained as described in Examples 8A and 8Aa through 8An:
Example 8Ba: ethane-1,2-dithiol
Example 8Bb: propane-1,3-dithiol
Example 8Bc: butane-1,4-dithiol
Example 8Bd: pentane-1,5-dithiol
Example 8Be: hexane-1,6-dithiol
Example 8Bf: 1,4-benzenedithiol
Example 8Bg: 1,3-benzenedithiol
Example 8Bh: 1,4-benzenedimethanethiol
Example 8Bi: 1,3-benzenedimethanethiol
Example 8Bj: 1,2-benzenedimethanethiol

### Example 8C: PEG-diamine copolymer with pendant alkyl groups

By the method of example 6B, the substituted PEG succinate obtained as described in Example 8A is co-polymerized with 1,4-diaminobutane.

By this method, the following diamines are co-polymerized with any of the substituted PEG succinates of Examples 8A and 8Aa through 8An:
Example 8Ca: *2O*-BOC 1,3-diamino-2-propanol
Example 8Cb: N', N"-di(BOC) hexaethylene tetraamine
Example 8Cc: N', N"-di(BOC) spermine
Example 8Cd: N'-BOC spermidine
Example 8Ce: N', N", N"'-tri(BOC) pentaethylene hexamine
Example 8Cf: agmatine
Example 8Cg: lysine t-butyl ester
Example 8Ch: 1,6-diaminohexane
Example 8Ci: 1,4-phenylenediamine
Example 8Cj: 1,3-phenylenediamine
Example 8Ck: 1,4-diaminobutane-2,3-diol acetonide

### Example 9: PEG Trans-esterifrcation Using Substituted Acids

PEG ditosylate: To 1 mol of PEG (dissolved in DMF or melted as is) was added 2.1 mol of tosyl chloride (5% molar excess) while stirring under argon. To this reaction mixture was added 2.2 mol of tetramethyl ethylene diamine (TEMED). The reaction was then incubated at 45 °C for 2h. The products were resolved using TLC in ethylacetate, toluene, or ethanol as TLC solvents. The PEG ditosylate may be extracted from the reaction mixture with toluene. Instead of tolunesulfonyl chloride, other sulfonylating agents such as mesyl chloride (see Example 4), triflic anhydride, or tresyl chloride may also be used (see U.S. Patent Application 10/397332, Publication No. 20040006051).

Polyesterification of PEG ditosylate: To 1 mol of molten PEG-ditosylate, with stirring under argon, is added 1 mol of S,S'-didecyl-*meso*-2,3-dimercaptosuccinic acid and 2 mol of TEMED. DMF is added as necessary to maintain fluidity. The reaction mixture is heated to 80° C and stirred for 24 h or until complete by TLC.

### Example 10: PEG-Di(succinyl)-di-(4-Acylated)thioether Medium Molecular Weight Polymer (C16-π-Polymer B)

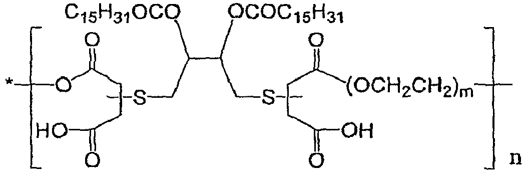

PEG-dimaleate (10.24 g, 6.1 mmols) prepared as in Example 1 was placed in a dry 125 ml flask and heated to 70 °C under argon to melt the PEG-dimaleate. To this molten material, with stirring, was added water (10 mL) and a solution of DTT (0.961 g, 6.168 mmols) and TEMED (0.723 g, 6.166 mmols) in water (3 mL). The solution was stirred at 70 °C for about 4 hr. Removal of water *in vacuo* gave the solid polymer in about 90% yield.

The dried polymer (5 g, 2.7 mmols) was heated to 70-90 °C under argon to melt it, and TEMED (0.635 g, 5.5 mmols) was added. Palmitoyl chloride (1.689 g, 5.5 mmols) was added with stirring, and the mixture was stirred under argon overnight. (The ratio of polymer to acyl chloride can be varied to obtain degrees of substitution from 0-100% of stoichiometry.) Water was added to the reaction mixture to isolate the "C16-π-Polymer B".

By this method the following acids are esterified with the hydroxyl groups of the di(succinyl)PEG-DTT copolymer:
Example 10a: Oleic acid
Example 10b: Cholesteryl succinate
Example 10c: Biphenyl-4-carboxylic acid
Example 10d: 4-Octylphenylacetic acid
Example 10e: Hexadec-6-ynoic acid

As an alternative to the use of acid halides, the DTT-derived hydroxyl groups of π-polymers may also be activated with 1,3-bis (2,2-dimethyl-1,3-dioxolan-4-ylmethyl) carbodiimide (BDDC) and coupled directly with carboxylic acids; see Handbook of Reagents for Organic Synthesis, Reagents for Glycoside, Nucleotide, and Peptide synthesis, Ed. David Crich, Wiley, 2005 p 107-108 and references therein).

### Example 11: Carboxyl substituted esters of C16-π-Polymer A.

Carboxylic acid-substituted polymers are used to attach ligands having reactive amino groups, using standard peptide bond formation methodologies (e.g., via carbodiimide reagents) to link the amino groups to the carboxylic acid functionality of the polymer. These materials are readily obtained by esterification of π-polymer hydroxyl groups with cyclic anhydrides. For example, C16-π-Polymer A dimaleate was prepared by reacting maleic anhydride with C16-π-Polymer A hydroxyl groups as follows:
*C16-π-Polymer A* (2 g) and maleic anhydride (0.85 g) were ground in a dry mortar and transferred to a 50 mL round bottom flask. The flask was heated at 90°C, under argon, for 2-3 hr with stirring. The solid reaction mixture was then ground and slurried with water, and the mixture was transferred to a dialysis bag (3.5 kDa cut-off). The mixture was dialyzed against water to remove excess maleic acid and low molecular weight by-products, and the retentate was removed from the bag and dried at 60°C to constant weight, to give *C16-π-Polymer A dimaleate* (1.79 g). The ratio of Polymer A to maleic anhydride can be varied to obtain substitutions varying from 0-100% of full stoichiometric esterification.

### Example 11a: C16-π-Polymer A diglycolate

*C16-π-Polymer A* (2 g) and diglycolic acid anhydride (1.0 g) were reacted by the method of Example 11 above, to give *C16-π-Polymer A diglycolate.* As with maleic anhydride, the ratio of Polymer A to anhydride can be varied to obtain substitutions varying from 0-100% of full stoichiometric esterification.

### Example 11b: C16-π-Polymer A bis(aconitate)

*C16-π-Polymer A* (2 g) and aconitic acid anhydride (1.35 g) were reacted by the method of Example 11 above, to give *C16-π-Polymer A bis(aconitate).*

In a similar manner, the following anyhydrides are coupled with *C16-π-Polymer A.* When using anhydrides of low solubility, the pH may be adjusted to between 4.5 and 6.5 prior to dialysis as an aid to purification. A second dialysis against 0.1 N HCl provides the acid form of the polymer, if desired.
Example 11c: succinic anhydride
Example 11d: glutaric anhydride
Ecample 11e: phthalic anhydride

The reactive double bond introduced through esterification with maleic or *cis*-acotinic anyhydride may also be used to add thiol-containing ligands to the polymer, as described in Example 12 below.

### Example 12: Cysteine adduct of C16-π-Polymer A Dimaleate:

Powdered C16-π-Polymer A dimaleate (Example 11) (253 mg) was added to water (5 mL) and the mixture was stirred vigorously. Cysteine (24 mg) and TEMED (30.5 ul) were added to the reaction mixture, and the mixture was stirred at room temperature under an argon atmosphere. The progress of the reaction was monitored by TLC (silica gel plates, n-butanol-acetic acid-water, 3:1:1) with detection with ninhydrin. The reaction mixture showed a ninhydrin-positive spot co-migrating with the polymer. Cysteine also gave a ninhydrin-positive spot, whereas the starting polymer did not give any color with ninhydrin.

The method described above was used to introduce additional carboxyl groups for use as attachment points, using thiols having multiple carboxyl substituents. For example, mercaptosuccinic acid was added to the following C16-π-Polymer A diesters:
Example 12a: C16-π-Polymer A dimaleate
Example 12b: C16-π-Polymer A dicrylate
Example 12c: C16-π-Polymer A (bis)aconitate

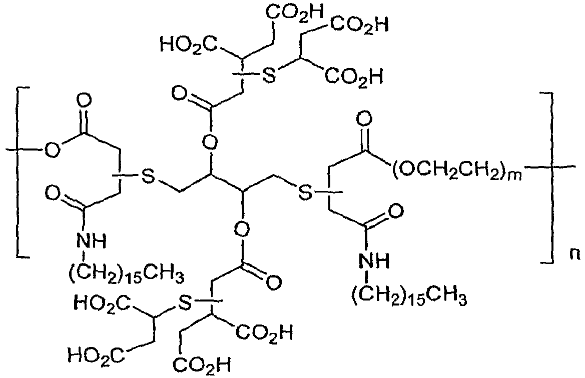

### Example 12c

In a similar manner, 3-mercaptoglutaric acid is added to the following C16-π-Polymer A diesters:
Example 12d: C16-π-Polymer A dimaleate
Example 12e: C16-π-Polymer A diacrylate
Example 12f: C16-π-Polymer A (bis)aconitate

### 3. Use of π Polymers to Solubilize Insoluble or Weakly Soluble Substances

### Comparative Example 1: Solubilization of Dyes

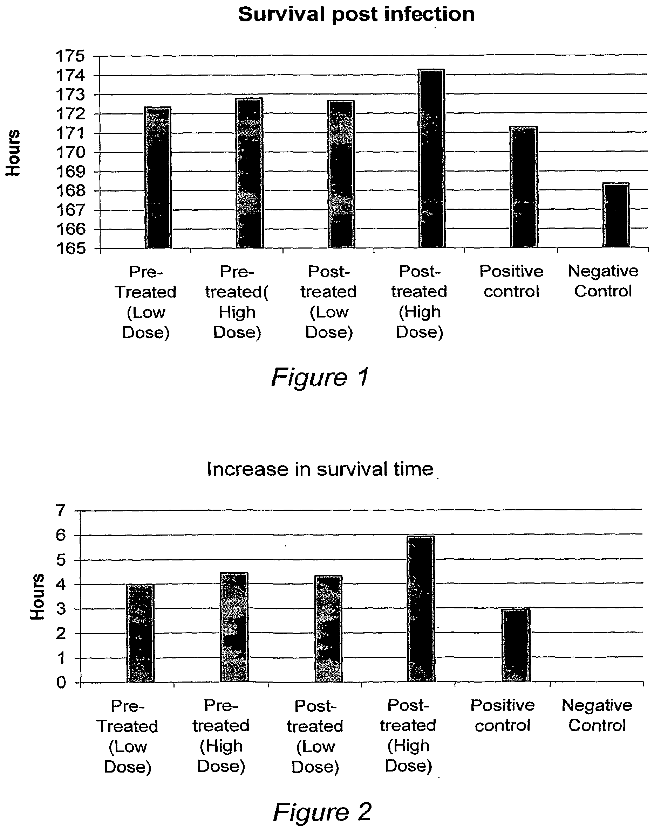

To 1.0 ml aliquots of a 50mg/ml aqueous solution of PEG1500-co-succinyl-DTT-bis-C16-amide polymer (C16-Polymer A, Example 1), centrifuged to remove insoluble materials but not otherwise purified, were added excess amounts of the dyes Eosin Y, dichlorofluorescein, and Sudan IV, in separate containers (FlexExcel™ clear polypropylene weigh-boats, WB2.5 size, product of AllExcel, Inc., West Haven, CT), and the components were stirred together to form a paste. The container bottoms were then attached to the bottom of a small jewelry ultrasonic cleaner bath using a water-resistant double-stick tape. Just enough water was added to the bath to immerse the weigh boats to about 1/3rd height. Sonication was performed for 15 minutes in steps of 5 minutes. The liquids were transferred to centrifuge tubes and centrifuged twice for 30 min. in a bench top centrifuge to pellet out undissolved dye. The supernatants were transferred to clean tubes and centrifuged again, to remove entrained solids. Suspensions of same amounts of dyes in same amount of distilled water as the amount of the polymer solution were treated in the same fashion, as controls. The resulting solutions were spotted (25 ul) on TLC plates to form circles from the drops. The intensities of the spots were compared with spots made from standards of dye solutions made in ethanol or ethanol/water to determine approximate concentrations; the spots are shown in Figure 1. The solubilities of the dyes in water were determined by dissolving appropriate amount of the dye in 1 1 or more deionized water (unbuffered) at room temperature, and adding (i.e. titrating with) further water as necessary to obtain saturated solutions.

The concentration of Sudan IV in 50mg/ml polymer was approximately 0.2 mg/ml, as opposed to 0.000 mg/ml in H₂O (Sudan IV is insoluble at neutral pH). The concentration of Dichlorofluorescein was approximately 5mg/ml in 50mg/ml polymer, as opposed to 0.010 mg/ml in H₂O. The concentration of Eosin Y in 50mg/ml polymer was approximately 5 mg/ml, as opposed to 0.007 mg/ml in H₂O. The payload ratios (amount of drug per unit amount of polymer, g/g) were calculated to be approximately 1:250 for Sudan IV, 1:10 for dichlorofluorescein and 1:10 for Eosin Y.

The payload ratios of 1:10 for polar compounds that resemble pharmaceutically active substances in physicochemical properties are higher than those generally attainable with liposomes, cyclodextrins, Cremophor™, or detergent or other solubilizing systems. Eosin Y is a photoactivable singlet oxygen generator with a very high efficiency, and such concentrated solutions of Eosin Y as are made with the polymer of Example 1 may be expected to be pharmacologically active as photoactivable cytotoxic agents.

The change in fluorescence spectrum of dichlorofluorescein in the polymer solution (reddish yellow/orange) over that in water (greenish yellow) was visually noticeable and gives an indication that the dye is not in an aqueous environment, but is encapsulated in the organic environment of the self-assembled polymer particle cores. Indeed, changes in fluorescence spectra have been used as a method of determining changes in the polarity of the microenvironment (e.g. "lipid probes"). The color of the Sudan IV solution in the polymer was reddish brown, as opposed to red in ethanol solution and brown powder when suspended in water. Eosin Y did not show a significant visual shift (pink in water to reddish pink in the polymer solution).

### Comparative Example 2: Solubilization of Medically Relevant Substances

Purpurin, Amphotericin B, Camptothecin and Doxorubicin were selected as representative sparingly soluble active pharmaceutical ingredients (API). Amphotericin B is used in a liposomal formulation as an injectable antifungal, while Camptothecin and Doxorubicin are anticancer agents. Purpurin is a DNA intercalating dye with potential pharmaceutical utility, and Eosin Y is a photosensitive singlet oxygen reagent with potential use in photodynamic therapy. Each API was solubilized in water with C16-π-Polymer A, C18-π-Polymer B, and/or C16-π-Polymer A-folic acid conjugate (see below). Solubilization was demonstrated by spotting the solubilized API and non-solubilized controls on TLC plates, as described above for the dyes.

Dried polymers were reconstituted with water, with heating, agitation, and sonication as necessary. When the solution was too viscous, it was diluted. C16-π-Polymer A was used at 10%w/v, folated C16-π-Polymer A was used at 5% w/v, and C18-π-Polymer B was used at 2% w/v.

Drug substance (20 mg) was added directly to 1ml of polymer solution, resulting in polymer:API mass ratios of 5:1 for C16-π-Polymer A, 2.5:1 for folated C16-π-Polymer A, and 1:1 for C18-π-Polymer B, except for doxorubicin (see below). The mixtures were sonicated for 1 hr at low power, and then centrifuged twice at 2000 xg to remove undissolved solids. The amount of pelleted solids was not significant.

Doxorubicin hydrochloride was combined with polymers as above at a 10:1 C16-π-Polymer A to doxorubicin hydrochloride mass ratio, or at a 5:1 folated C16-π-Polymer A to doxorubicin mass ratio, followed by addition of sufficient 3M sodium acetate to neutralize the doxorubicin hydrochloride. The mixtures were vigorously shaken for 24 hours and then twice centrifuged at 2000 xg to remove undissolved solids. The amount of pelleted solids was not significant.

The mass ratios of solubilized APIs to polymer are shown in Table 1. No attempt was made to maximize the loading of the polymer, therefore these ratios represent lower limits on the amount of API the polymers are capable of carrying into solution.

A 10 ul sample of each solution was spotted on a Bakerflex™ silica gel TLC plate and allowed to spread. The aqueous solution forms an outer boundary of the circle and an inner circle formed by migration of the polymer with encapsulated material. In all cases, there was very little API in the peripheral fringe of the aqueous-only zone, indicating successful solubilization and minimal leakage of the encapsulated material.

**Table 1: Solubilization of APIs**

| Polymer:Substrate Mass Ratios | | | |
|---|---|---|---|
| | C16-π-PolymerA 10% w/v | Folated C16-π-Polymer A 5% w/v | C18-π-Polymer B 2% w/v |
| Purpurin | 5:1 | 2.5:1 | not done |
| Camptothecin | 5:1 | 2.5:1 | not done |
| Amphotericin B | 5:1 | 2.5:1 | not done |
| Doxorubicin | 10:1 | 5:1 | not done |
| Eosin Y | not done | not done | 1:1 |

### 4. Biocompatibility of π Polymers

### Comparative Example 1: Suitability for topical emollients, creams or pastes

A concentrated oily wax of the polymer of Example 1 was rubbed on the inner wrist skin by the inventor and observed for uptake. The material appeared to be absorbed similarly to pharmacological waxy creams, with slight softening of the area. No immediate or delayed allergic responses, such as reddening, rash, or itching, were observed upon this single topical application.

Many of these polymers are hygroscopic waxes at room temperature, with an expected mp of about 45 °C to 60 °C or greater, depending upon the composition. Polymers made with lower MW PEG's may even be liquid at room temperature. Some polymers may be solid at room temperature, melting at body temperature. Thus the properties of these π polymers make them excellent substrates for making lotions, creams, ointments, emollients, and other delivery forms, either by themselves, or in mixture with various substances, including active pharmaceutical agents.

### Comparative Example 2: Suitability for parenteral administration

An aqueous solution of the polymer of Example 1 was prepared in phosphate-buffered saline and then filtered into sterile tubes through 0.22um filters.

A maximum tolerated dose protocol was employed, wherein CD-1 mice were subjected to a dose of 10ml per kg body weight tail vein injection of up to 5% w/v aqueous solution of the polymer. The mice were observed for 12 hours continuously and every 2hr thereafter until 48 to 72 hrs, depending upon the group. Blood samples were taken and analyzed. Some mice were sacrificed and first examined for gross histology. Microscopic histology was then performed on selected sections.

No observable differences were found in the blood chemistry between the control mice and the treated mice. No observable differences or lesions were found compared to control animals in the gross histology of various organs including heart, lungs, kidneys, spleen, liver, intestines, stomach, bladder, skin, muscles, bones, brain, and lymph nodes. Multiple specimens from different groups of animals were studied with the same results being observed. No obervable differences were found in cellular tissue structure of examined tissues. Some of the kidneys showed some casting that diminished with exposure time to the polymer. This implies that the casting is a temporary phase and as the time progresses it will become normal.

It is concluded that the polymer is safe for medical use as a pharmaceutical agent in injectable preparations and other parenteral formulations. It is reasonable to expect that the polymer is safe in oral solutions, caplets, and tablets, nasal spray, oral/bronchial aerosols, sublingual, skin cream/lotion/patch, eyedrops, other topical routes, and other routes of administration.

### 5. Attachment of Targeting Moieties to π-Polymers

### Comparative Example 1: Attachment ofgalactosamine to C-16 π-Polymer B via amide bond formation.

Galactosamine (GA) targets the hepatic asialoglycoprotein receptor (ASGPR), and polymers bearing covatently-bonded glactosamine are delivered to the liver; see L. Seymour et al., "Hepatic Drug Targeting: Phase I Evaluation of Polymer-Bound Doxorubicin" J. Clin. Oncology, 20(6): 1668-1676 (2002) and references therein.

C16-π-Polymer B (Example 10 in synthetic method section above) (461 mg, 0.2 mmols equivalent COOH per repeating unit) was dispersed in 14 mL water, and to this dispersion was added EDC HCl (0.485 mmols) and N-hydroxysuccinimide (0.464 mmols). The mixture was stirred at ambient temperature for 15 minutes and a solution of galactosamine HCl (0.386 mmols) and TEMED (0.387 mmols) in 1 ml water was added. The solution was stirred and the reaction was followed by TLC on silica gel and development in 1-butanol-acetic acid-water (3:1:1). An additional amount of TEMED (0.079 mmols), NHS (0.078 mmols) and EDC HCl (0.193 mmols) were added to force the reaction to completion. When TLC showed a steady state with respect to consumption of GA, the reaction mixture was dialyzed (3500 Da cut-off membrane) against 3 x 1000 ml deionized water to remove the low molecular weight reactants and by-products. The retentate was removed and dried at 60°C to constant weight (348 mg).

TLC of the product showed no free GA (ninhydrin negative). A sample of the product was hydrolyzed with 6 N HCl at 100°C to hydrolyze bound GA. TLC analysis showed the presence of GA (ninhydrin positive) at the same Rf as reference GA.

### Comparative Example 2: Attachment of folic acid to C18-π-Polymer A

BDDC (2.44 g, 8.56 mmols) was weighed out in a 125 mL round-bottom flask flushed with argon (BDDC is very viscous with honey like consistency and difficult to handle). C18-π-Polymer A (10 g, 4.28 mmols) was added to the flask, the mixture was heated to 70°C, and the reactants were stirred together for about 30 minutes. Folic acid (3 g) was added followed by sufficient THF to make stirring possible. The reactants were stirred at 40-70°C overnight, protected from moisture. The THF was then allowed to evaporate and water (80 mL) was added, and the mixture was stirred at 50 °C for an additional 2 h. After cooling to room temperature, the mixture was transferred to a section of dialysis tubing with a 3500 Dalton cut off, and dialyzed against 0.1 N HCl (2 x 2000 ml), water (2000 ml), 5% sodium carbonate (2 x 2000 ml) and water (4 x 2000 ml), to remove unreacted reagents and by-products. The bright yellow-orange retentate was was removed. A portion was evaporated to constant weight to determine the solid concentration, and was used for the solubilization experiments described above.

### Example 3: Attachment of N-acetyl neuraminic acid (NANA) and analogues to π-Polymers.

Neuraminic acid derivatives are targeting moieties for influenza viruses because of the hemagglutinin and neuraminidase coat proteins, both of which are known to bind to sialic acid. Several methods for coupling NANA and its derivatives to the π-polymers of the invention were developed.

### Example 3a: Attachment of N-acetyl neuraminic acid (NANA) to C16-π-Polymer A via esterification.

BDDC (2.44 g, 8.56 mmols) and C18-π-Polymer A (10 g, 4.28 mmols) are combined and heated to 70 °C, and stirred together under argon for about 30 minutes. N-acetyl neuraminic acid (3 g) is added, followed by THF as necessary to maintain fluidity. The reactants are stirred at 40-70°C overnight, protected from moisture. Water (80 mL) is added, and the mixture is stirred at 50 °C for an additional 2 h. After cooling to room temperature, the mixture is dialyzed against 0.1 N HCl, 5% NaHCO₃, and water (2 x 2000 ml each) with a 3.5 kDa cutoff membrane. Spotting on a silica gel TLC plate, and visualization with 0.2% orcinol in 70% sulfuric acid, at 130°C, shows incorporation of neuraminic acid into the polymer.

### Example 3b: Attachment of N-acetyl neuraminic acid (NANA) monomaleate to C16-π-Polymer A.

5-N-Acetylneuraminic acid (NANA) (0.86 mmols), maleic anhydride (0.93 mmols) and triethylamine (1.77 mmols) were dissolved in 1.5 mL DMSO in a dry round bottomed flask. The flask was flushed with argon and placed in an oil bath. The mixture was stirred at 65° C to 85°C and the progress was checked by TLC on silica plates (i-PrOH-EtOAc-water, 4:3:2) until the reaction was complete (absence of NANA, detection with orcinol /H₂SO₄ or urea/HCl reagent). The reaction mixture was cooled to room temperature, and water was added to hydrolyze the excess maleic anhydride. The resulting solution of NANA monomaleate was used directly in subsequent reactions.

An aqueous solution of C16-π-polymer A diglycolate (See "Synthesis of π-polymers", example 11a) (1.23 mmols repeat units, 2.46 mmols -COOH) was adjusted to pH 4.5-6.5. Carbodiimide (EDC HCl, 3.86 mmols) and N-hydroxysuccinimide (2.6 mmols) were added and the mixture stirred at ambient temperature for about 60 min. A solution of NANA monomaleate (2.49 mmoles), prepared as described above, was added, and the pH was adjusted with TEMED to pH 6-7. Stirring was continued at ambient temperature for up to 26 hr. The product was purified by dialysis, first against 20 mmolar sodium acetate, pH 4.5 then against water. The retentate was removed and stored for use.

### Example 3c: Attachment of N-acelyl neuraminic acid (NANA) to C16-π-Polymer A, via a spacer.

Cysteamine (2-aminoethanethiol) hydrochloride (0.93 mmol in water) was added to an equimolar amount of NANA monomaleate (solution prepared as described above), followed by an equimolar quantity of TEMED to facilitate the addition of thiol to the double bond. The reaction was followed by TLC on silica (i-PrOH-EtOAc-water, 4:3:2) until the reaction was complete (absence of O-maleoyl-NANA, detection with orcinol /H₂SO₄ or urea/HCl reagent) to give targeting moiety **D**.

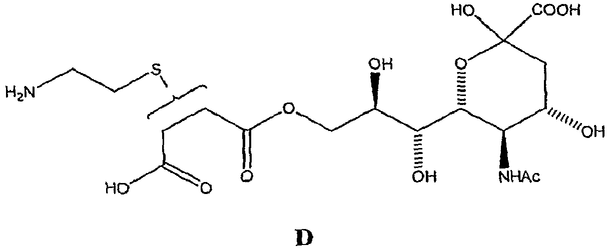

By the same method, 5-N-Acetyl-2,3-dehydro-2-deoxyneuraminic acid (DANA) was derivatized to give the targeting moiety **E**.

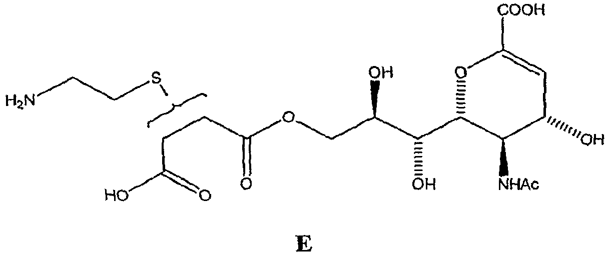

By the same method, cysteine and glutathione are added to the maleic acid monoesters of NANA and DANA.

By the method described in example 3b above, the mercaptosuccinate conjugate of C16-π-Polymer A bis(aconitate) was amidated with targeting moiety **D**. This polymer contained up to 8 -COOH groups per repeat unit (See "Synthesis of π-polymers", example 12c).

### Example 3d: Attachment of N-acetyl neuraminic acid (NANA) to C16-π-Polymer A, via a spacer.

By the method described above, targeting moiety **E** was conjugated to C16-π-polymer A diglycolate (See "Synthesis of π-polymers", example 11a)polymer.

### Example 3e: Attachment of neuraminic acid β-methylglycoside (MNA) to C16-π-Polymers.

having on average a single carboxyl per repeat unit (0.396 mmol) was dissolved in water and allowed to react with NHS (0.4 mmol) and EDC•HCl (0.64 mmol). Neuraminic acid-β-methylglycoside (MNA) (0.42 mmol) was added. The reaction mixture was stirred at ambient temperature (25-30 °C) for 18-24 h and then purified by dialysis.

### Example 3f:

A second sample of C16-π-polymer A diglycolate having two carboxyl groups per repeat unit was also conjugated with MNA in the same fashion.

### Example 3g: Attachment of β-O-methyl neuraminic acid (MNA) to C16-π-Polymer B.

C16-π-Polymer B, 43 micromoles COOH basis, in 1 ml water, and neuraminic acid β-methyl glycoside (Toronto Research Chemicals), 40 micromoles, were mixed together, and 40 micromoles NHS in 0.1 ml water was added, followed by 40 micromoles EDC hydrochloride in 0.1 ml water. The reaction mixture was shaken at ambient temperature for 48 hours, and analyzed by TLC on silica gel with isopropanol-ethy acetate-water (4:3:2). Detection with 0.2% orcinol in 70% sulfuric acid, at 130°C, does not generate a color reaction with the starting polymer, but TLC of the reaction mixture gave a purple spot co-migrating with the polymer.

All polymer conjugates in the above examples (3a-3g) show a positive reaction, after dialysis, for the presence of neuramininic acid when visualized with orcinol/sulfuric acid or urea/HCL reagent on TLC.

### Example 4: Attachment of zanamivir to C16-π-Polymer B.

Zanamivir (GG167) is a potent inhibitor of viral neuraminidase, and polymers bearing this molecule as a multivalent ligand are inhibitors of influenza virus replication.

C16-π-Polymer B (920 mg) is dispersed in 30 mL water, and to this is added EDC HCl (1.2 mmol) and N-hydroxysuccinimide (1.1 mmol). The mixture is stirred at ambient temperature for 20 minutes, and a solution of the trifluoroacetic acid salt of 5-acetamido-7-(6'-aminohexyl)-carbamyloxy-4-guanidino-2,3,4,5-tetradeoxy-D-glycero-D-galacto-non-2-enopyranosonic acid (U.S. Patent Nos. 6,242,582 and 6,680,054) (0.39 g, 0.67 mmol) and TEMED (0.67 mmols) in 1 ml water is added. The solution is stirred at room temperature, and the reaction is followed by TLC. The reaction mixture is dialyzed (3500 kDa cut-off membrane) against 3 x 1000 ml deionized water to remove the low molecular weight reactants and by-products. The retentate is removed and dried at 60°C to constant weight. The level of sugar incorporation may be determined by a colorimetric assay for the guanidine group (Can.J. Chem., 36:1541 (1958)). A neuraminidase assay may be carried out following the procedure of Potier et al, Anal. Biochem., 29 287 (1979).

### Example 5: Attachment of Mimosine.

C16-π-polymer A diglycolate (See "Synthesis of π-polymers", example 11 a) as a 4.5 % w/v solution (1 mmol repeat units, ca. 2 mmol in COOH groups) was reacted with NHS (2.27 mmol) and EDC•HCl (2.23 mmols), and to the resulting mixture a solution of 1-mimosine (2.14 mmol, prepared in 5 ml water and pH adjusted with TEMED to increase the solubility) was added and stirred at ambient temperature and pH of about 6.8-7 for about 22-24 h. The pH was adjusted to3-4 with 6 N HCl, the mixture stirred for 15-30 min, and the pH raised to 6.1 by addition of TEMED. The mixture was then dialyzed (3.5 kD cutoff) against water to remove impurities and low molecular weight products.

### Example 6: Attachment of peptides and proteins to π-Polymer A Dimaleate and Diacrylate:

General procedure for Fab fragments: Disulfide bonds in antibody F(ab')2 fragments are reduced with immobilized TCEP Disulfide Reducing Gel (Pierce, Product # 0077712) using the manufacturer's protocol; or alternatively with DTT or TCEP in solution, the spent reagents being removed by ultrafiltration using 30kD filters. The reduced F(ab')2 fragments containing free sulfhydryl groups are then reacted with a π-Polymer A dimaleate or diacrylate in the presence of TEMED.

General procedure for cysteine and cysteine-containing peptides: The acrylate or maleate ester of a π-polymer A is reacted with cysteine residues using triethylamine as catalyst. To a polymer diacrylate (0.3 mmols repeat unit, 0.6 mmols acrylates) suspension in water was added cysteine (0.66 mmol) and triethylamine (1.32 mmol). The flask was flushed with argon and stirred at ambient temperature overnight (about 18 h). TLC of the reaction mixture on silica (i-PrOH-Ethyl acetate-water, 4:3:2) showed the absence of cysteine and a ninhydrin-positive spot for the polymer, indicating addition of cysteine to the acrylate double bonds.

### Example 6a: Attachment of anti-rabies antibody fragments to C16-π-Polymer A Diglycolate:

C16-π-Polymer A dimaleate was prepared, starting with PEG of molecular weight 4500. BayRab™ human rabies immunoglobulin (hIgG) was treated with pepsin in the usual manner in an acidic pH buffer to generate the F(ab')2 fragment, which was purified by ultrafiltration using 50kD filters. The F(ab')2 fragment was coupled to the PEG 4500 C-16-π-Polymer A diglycolate by the EDC method described in Example 5 above.

### Example 6b: Attachment of anti-rabies antibody fragments to C16-π-Polymer A Dimaleate:

The F(ab')2 fragment of BayRab™ hlgG (see example 6a) was reduced with DTT (or alternatively TCEP), and spent reagent was removed by ultrafiltration using 30kD filters. The Fab'-SH fragments were coupled to PEG 4500 C-16-π-polymer A dimaleate by Michael addition of the free thiol to the maleic acid double bond at pH 7-8.3 (TEMED). The conjugates were purified by ultrafiltration using 100kD filters to remove low molecular weight contaminants.

Example 6c: PEG 8500 C-16-π-polymer A dimaleate was conjugated to reduced F(ab')2 fragment of BayRab™ hIgG as described above.

### Example 6d: Attachment of Peptides toC16-π-Polymer A Dimaleate:

The peptide KDYRGWKHWVYYTC ("Rab 1") has been reported to bind to Rabies virus (T.L. Lentz, 1990, J. Mol. Recognition, 3(2):82-88). The terminal Cys of this peptide was used to synthesize an Anti-Rabies Peptide-π-Polymer A conjugate. The C16-π-polymer A dimaleate (two maleic acid moieties per repeat unit) derived from PEG 1500 (0.157 mmol) was dissolved in water (6 mL) and the pH of the solution was adjusted to about 8 with TEMED. The peptide (0.157 mmol) dissolved in DMF (3.1 ml) was added, and the reaction mixture was stirred at ambient temperature under argon while the reaction pH was maintained between 8-8.3. Progress of the reaction was checked by testing the reaction mixture with Ellman's reagent. After about 45 h, the Ellman's test was almost negative. Water was added to bring down the DMF concentration and the reaction mixture was centrifuged to remove a small amount of precipitate. The clear supernatant was ultrafiltered through a 10 kD centrifugal filter unit (Amicon Ultra 10 kD, cat# UFC901024) and the retentate washed with water repeatedly to remove low molecular weight contaminants.

The following three peptides (O = ornithine; NH₂ designates a C-terminal amide) were prepared by automated solid-phase synthesis, and conjugated to PEG1500 π-polymer A dimaleate in the same manner as the Rab1 peptide:
Example 6d: KDYRGWKOWVYYTC ("Rab2")
Example 6e: KGWKHWVYC(NH₂) ("Rab3")
Example 6f: KGWKOWVYC(NH₂) ("Rab4")

### 6. Antiviral activity of π-polymers.

### Example 1: Efficacy against Influenza.

ATCC VR-1520 (H2N1) Human influenza virus was used in a mouse protection assay. A single tail vein injection, 200ul/20g BW led to 99.5% lethal infection in control animals (7 days untreated survival).

Ten mice were in each group. Mice were tail-vein-injected with 200ul/20g body mass of a low dose (0.0375%) and a high dose (0,15%) solution of the π-polymer B-MNA conjugate of Example 3 above. Post-treated animals were dosed 24 hours after infection, while pre-treated animals were dosed 6 hours prior to infection. Positive control animals were injected with an equivalent amount of free ligand, while the negative controls received a saline buffer injection.

The survival time was used as the index efficacy end point. Body weight was tracked as a study parameter. Histology of internal organs was performed in both gross examination and microscopic examination. Results are shown in Figure 2.

The increase in survival time was 5.93 hours (+/-0.48h SD) for the high dose treatment group, compared to only 2.94 hours (+/- 0.75h SD) for the positive control (Figs. 1 and 2). On the basis of the mass of ligand, the high dose treatment corresponds to, at most, 0.03% of the ligand, assuming the maximum polymer-conjugate substitution ratio of 0.2 w/w. Thus the polymer conjugate showed a significantly high level of efficacy compared to the unconjugated ligand control.

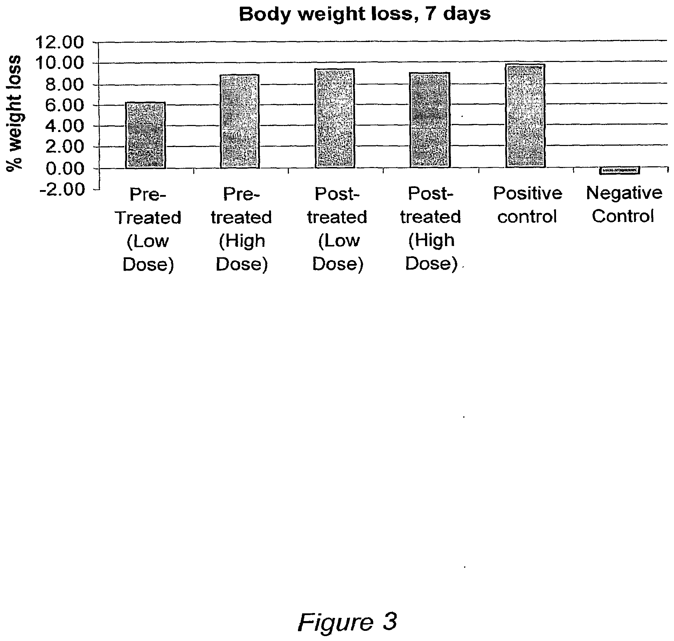

Gross histology as well as microscopic examination of some mice in the high dose polymer B-MNA conjugate treatment group showed normal patterns, except that in bone marrow sections the treated mice showed slightly reduced level of white cells, which may be attributable to the effects of the influenza infection. The body weight loss in protection groups (high dose - 8.9%, low dose - 6.2%) as well as treatment groups (high dose - 9.0%, low dose - 9.4%) was less than that in the positive control (- 9.8%), suggesting an association with the ligand itself rather than the polymer (Fig. 3). A small weight gain of 0.7% occurred in untreated mice.

### Example 2: Efficacy against Rabies.

Groups of ten White Swiss mice, ca. 20 g each, mixed sexes, were employed in an in vivo protection assay. Mice were challenged by injection of 3x the LD50 of rabies virus. Injections were 0.03 ml CVS (challenge virus standard) rabies strain, at a dilution of 10⁻⁶ (100 LD₅₀/ml). Day-to-day survival, paralysis, and body mass were monitored. Intraperitoneal administration of drug at 25, 48, and 72 hr, and intracerebral administration of drug at 25 and 48 hr, were investigated. Results are presented in Tables 1 and 2.

**Table 1**

| **Experimental Rabies in Mice** | | | | | | | | | | | |
|---|---|---|---|---|---|---|---|---|---|---|---|
| **Intraperitoneal administration; number of survivors** | | | | | | | | | | | |
| **Example # (dose/inj., mg)** | **Days post-infection** | | | | | | | | | | |
| | **1*** | **2*** | **3*** | **4** | **5** | **6** | **7** | **8** | **9** | **10** | **11** |
| 6a (0.4) | 10 | 10 | 10 | 10 | 10 | 10 | 4 | 1 | 1 | 1 | 0 |
| 6b (2.0) | 10 | 10 | 10 | 10 | 10 | 10 | 5 | 3 | 0 | | 0 |
| 6c (2.0) | 10 | 10 | 10 | 10 | 10 | 10 | 7 | 1 | 0 | | 0 |
| 6d (2.1) | 10 | 10 | 10 | 10 | 10 | 10 | 5 | 1 | 1 | 1 | 0 |
| 6e (2.4) | 10 | 10 | 10 | 10 | 10 | 10 | 5 | 0 | | | 0 |
| 6f (3.0) | 10 | 10 | 10 | 10 | 10 | 10 | 5 | 1 | 1 | 1 | 0 |
| 6g (2.6) | 10 | 10 | 10 | 10 | 10 | 10 | 4 | 0 | | | 0 |
| Rab1 peptide (0.5) | 10 | 10 | 10 | 10 | 10 | 10 | 7 | 2 | 0 | | 0 |
| Rab2 peptide (0.5) | 10 | 10 | 10 | 10 | 10 | 10 | 5 | 3 | 1 | 1 | 0 |
| Rab3 peptide (0.5) | 10 | 10 | 10 | 10 | 10 | 10 | 5 | 1 | 0 | | 0 |
| Rab4 peptide (0.5) | 10 | 10 | 10 | 10 | 10 | 10 | 3 | 0 | | | 0 |
| 6d (1.0) | 10 | 10 | 10 | 10 | 10 | 10 | 5 | 1 | 1 | 1 | 0 |
| 6e (1.2) | 10 | 10 | 10 | 10 | 10 | 10 | 4 | 1 | 1 | 0 | 0 |
| 6f (1.5) | 10 | 10 | 10 | 10 | 10 | 10 | 5 | 0 | | | 0 |
| 6g (1.3) | 10 | 10 | 10 | 10 | 10 | 9 | 4 | 1 | 0 | | 0 |
| BavRab™ (0.4) | 10 | 10 | 10 | 10 | 10 | 10 | 5 | 0 | | | 0 |
| BavRab™ (2.0) | 10 | 10 | 10 | 10 | 10 | 10 | 6 | 1 | 0 | | 0 |
| 6b (0.4) | 10 | 10 | 10 | 10 | 10 | 9 | 3 | 0 | | | 0 |
| 6c (0.4) | 10 | 10 | 10 | 10 | 10 | 10 | 4 | 0 | | | 0 |
| | | | | | | | | | | | |
| Saline | 10 | 10 | 10 | 10 | 10 | 10 | 6 | 2 | 0 | | 0 |
| None | 10 | 10 | 10 | 10 | 9 | 9 | 5 | 1 | 1 | 1 | 0 |

| | | | | | | | | | | | |
|---|---|---|---|---|---|---|---|---|---|---|---|
| *doses injected on days 1-3 | | | | | | | | | | | |

**Table 2**

| **Intracerebral administration; number of survivors** | | | | | | | | | | | |
|---|---|---|---|---|---|---|---|---|---|---|---|
| **Example # (dose/inj., mg)** | **Days post-infection** | | | | | | | | | | |
| | **1*** | **2*** | **3** | **4** | **5** | **6** | **7** | **8** | **9** | **10** | **11** |
| BayRab™ (0.4) | 9 | 9 | 9 | 9 | 9 | 9 | 9 | 7 | 1 | | 0 |
| 6g (0.4) | 10 | 10 | 10 | 10 | 10 | 10 | 7 | 3 | 2 | 2 | 0 |
| Saline | 10 | 10 | 10 | 10 | 10 | 10 | 7 | 1 | 1 | 1 | 0 |

| | | | | | | | | | | | |
|---|---|---|---|---|---|---|---|---|---|---|---|
| *doses injected on days 1 and 2 | | | | | | | | | | | |

## Claims

1. A comb polymer consisting essentially of the following structure: 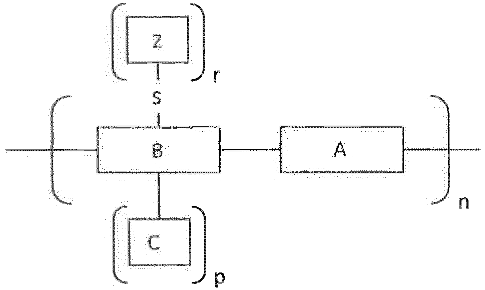 comprising a backbone formed of alternating branch-point moieties B and hydrophilic, water-soluble polymer blocks A; and having hydrophobic side chains C and optional ligands Z attached to the branch-point moieties, wherein:
the water-soluble polymer block A is selected from the group consisting of poly(ethylene glycol), poly(propylene glycol), poly(ethyleneimine), poly(vinyl alcohol), and poly(vinylpyrrolidone), and copolymers thereof;
the branch-point moiety B is a conjugate of dithiothreitol, dithioerythritol, or 2,3-diaminobutane-1,4-dithiol with two molecules of maleic acid;
each hydrophobic side chain C has a logP value greater than 1.4, and is independently selected from the group consisting of C₆-C₃₀ linear or branched hydrocarbons optionally substituted with one or more hydrophilic substituents; C₆-C₃₀ cyclic or polycyclic hydrocarbons optionally substituted with one or more hydrophilic substituents; and hydrophobic amino acids, peptides and polymers;
each ligand Z is independently a ligand having specific binding affinity for the surface of a virus;
s is a bond or a spacer moiety;
the value of n ranges from 3 to about 100;
the average value of p ranges from greater than one to four; and
the average value of r ranges from 1 to 4;
with the proviso that the comb polymer is not: 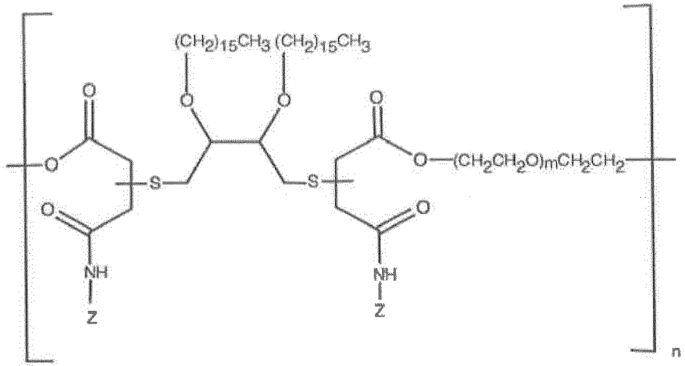 where -NH-Z is 6'-N-linked 5-acetamido-7-(6'-aminohexyl)-carbamyloxy-4-guanidino-2,3,4,5-tetradeoxy-D-glycero-D-galacto-non-2-enopyranosonic acid (zanamivir); 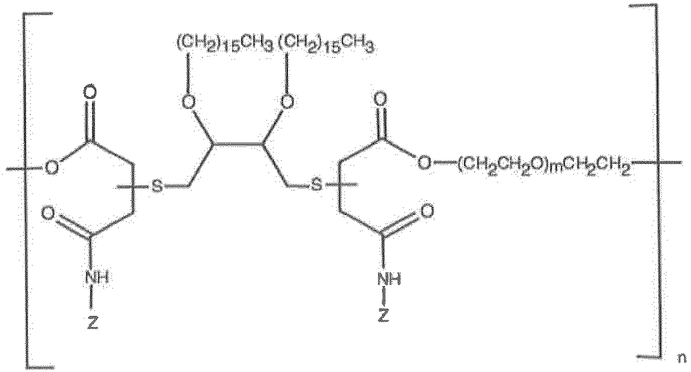 where -NH-Z is N-linked β-*O*-methyl neuraminic acid (MNA); or 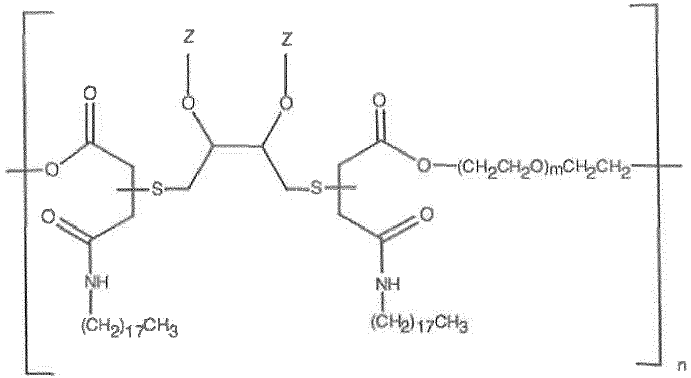 where Z is neuraminyl (NANA).

2. The comb polymer of claim 1, wherein at least one ligand is N-acetyl neuraminic acid, neuraminic acid β-methyl glycoside, or 4-guanidino-2,4-dideoxy-2,3-dehydro-N-acetylneuraminic acid.

3. The comb polymer of claim 1 wherein at least one ligand Z is a peptide, antibody or antibody fragment having specific binding affinity for the surface of said virus, wherein the ligand is preferably a Fab or F(ab')₂ fragment derived from human anti-rabies IgG immune globulin.

4. The comb polymer of claim 1, wherein at least one ligand Z is a peptide selected from the group consisting of KDYRGWKHWVYYTC, KDYRGWKOWVYYTC, KGWKHWVYC(NH₂), and KGWKOWVYC(NH₂).

5. The comb polymer of claim 1, wherein the polymer block A is selected from the group consisting of poly(ethylene glycol), poly(propylene glycol), and copolymers thereof.

6. The comb polymer of claim 5, wherein the polymer block A is poly(ethylene glycol), wherein the polymer block A has an average length ranging from 3 to 3,000 monomer units.

7. The comb polymer of claim 1, wherein the polymer has the structure 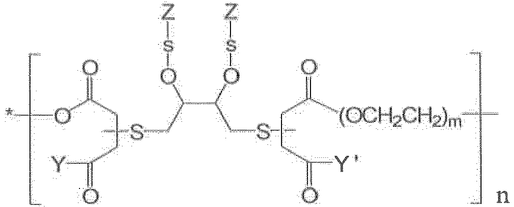 wherein m is 3-3,000, and Y and Y' are independently selected from the group consisting of R, OR, COOR, SR, NHR, NRR', ONHR, NHOR, NRNH₂, NHNHR, NRNHR', and NHNRR', wherein R and R' are independently selected from the group consisting of C₆-C₃₀ branched or linear hydrocarbons optionally substituted with one or more hydrophilic substituents, C₆-C₃₀ cyclic or polycyclic hydrocarbons optionally substituted with one or more hydrophilic substituents, and hydrophobic amino acids, peptides and polymers;
or
wherein the polymer has the structure 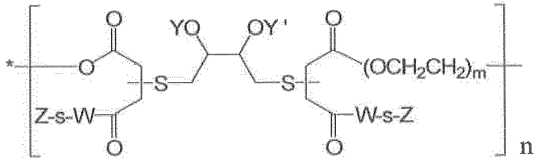 wherein m is 3-3,000, W is O or NH, and Y and Y' are independently selected from the group consisting of R, COR, COOR, CONHR, CONRR', CONHOR, CONRNH₂, CONHNHR, CONRNHR', and CONHNRR', wherein R and R' are independently selected from the group consisting of C₆-C₃₀ branched or linear hydrocarbons optionally substituted with one or more hydrophilic substituents, C₆-C₃₀ cyclic or polycyclic hydrocarbons optionally substituted with one or more hydrophilic substituents, and hydrophobic amino acids, peptides and polymers; or
wherein the polymer has the structure 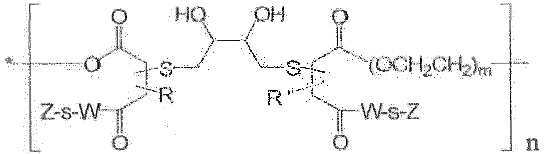 wherein m is 3-3,000, W is O or NH, and R and R' are independently selected from the group consisting of C₆-C₃₀ branched or linear hydrocarbons optionally substituted with one or more hydrophilic substituents, C₆-C₃₀ cyclic or polycyclic hydrocarbons optionally substituted with one or more hydrophilic substituents, and hydrophobic amino acids, peptides and polymers.

8. A peptide selected from the group consisting of KGWKHWVYC(NH₂), and KGWKOWVYC(NH₂).

9. Comb polymer according to any one of claims 1-7 for use in the treatment or prevention of viral infection.

10. The comb polymer according to any one of claims 1-2 and 5-7 for use in the treatment or prevention of an influenza virus infection.

## Patentansprüche

1. Ein Kammpolymer, im Wesentlichen bestehend aus der folgenden Struktur: 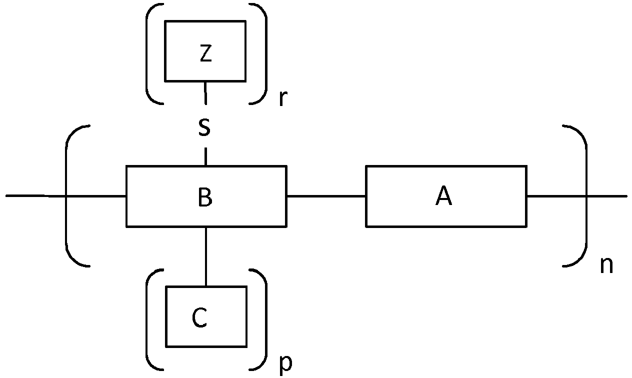 umfassend ein Rückgrat, das aus alternierenden Verzweigungsstelleneinheiten B und hydrophilen, wasserlöslichen Polymerblöcken A; und aus hydrophoben Seitenketten C und optionalen Liganden Z, die an die Verzweigungspunkt-Einheiten gebunden sind, gebildet ist, wobei:
der wasserlösliche Polymerblock A ausgewählt ist aus der Gruppe bestehend aus Poly(ethylenglykol), Poly(propylenglykol), Poly(ethylenimin), Poly(vinylalkohol) und Poly(vinylpyrrolidon) und Copolymere davon;
die Verzweigungsstelleneinheit B ein Konjugat aus Dithiothreitol, Dithioerythritol oder 2,3-Diaminobutan-1,4-dithiol mit zwei Molekülen Maleinsäure ist;
jede hydrophobe Seitenkette C hat einen logP-Wert von größer als 1,4 hat und unabhängig ausgewählt ist aus der Gruppe, bestehend aus linearen oder verzweigten C₆-C₃₀-Kohlenwasserstoffen, die gegebenenfalls mit einem oder mehreren hydrophilen Substituenten substituiert sind; C₆-C₃₀ cyclische oder polycyclische Kohlenwasserstoffe, die gegebenenfalls mit einem oder mehreren hydrophilen Substituenten substituiert sind; und hydrophobe Aminosäuren, Peptide und Polymere;
jeder Ligand Z ein unabhängiger Ligand ist, der eine spezifische Bindungsaffinität für die Oberfläche eines Virus hat;
S eine Bindung oder eine Spacer-Einheit ist;
der Wert n im Bereich von 3 bis etwa 100 liegt;
der Durchschnittswert von p im Bereich von größer als eins bis vier liegt; und
der Mittelwert von r im Bereich von 1 bis 4 liegt;
mit der Maßgabe, dass das Kammpolymer nicht ist: 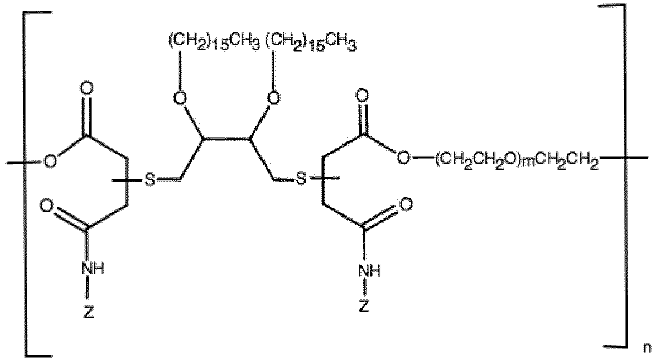 wobei -NH-Z 6'-N-verknüpftes 5-Acetamido-7-(6'-aminohexyl)-carbamyloxy-4-guanidino-2,3,4,5-tetradeoxy-D-glycero-D-galacto-non-2-enopyranosonsäure (Zanamivir) ist; 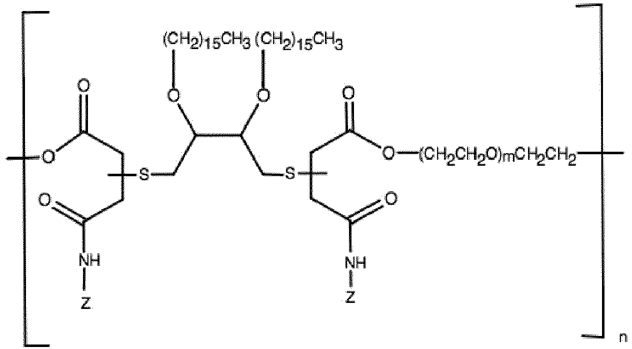 wobei -NH-Z N-verknüpftes β-*O*-Methylneuraminsäure (MNA) ist; oder 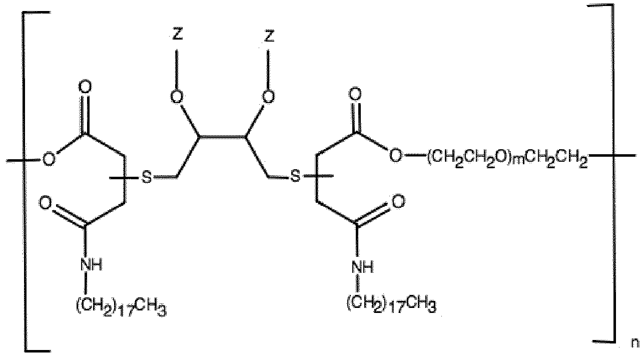 wobei Z Neuraminyl (NANA) ist.

2. Das Kammpolymer nach Anspruch 1, wobei mindestens ein Ligand N-Acetylneuraminsäure, Neuraminsäure-β-methylglycosid oder 4-Guanidino-2,4-dideoxy-2,3-dehydro-N-acetylneuraminsäure ist.

3. Das Kammpolymer nach Anspruch 1, wobei mindestens ein Ligand Z ein Peptid, ein Antikörper oder ein Antikörperfragment mit einer spezifischen Bindungsaffinität für die Oberfläche des Virus ist, wobei der Ligand vorzugsweise ein Fab oder F(ab')2-Fragment, das von einem humanen Anti-Tollwut IgG Immunglobulin abgeleitet ist.

4. Das Kammpolymer nach Anspruch 1, wobei mindestens ein Ligand Z ein Peptid ist, ausgewählt aus der Gruppe bestehend aus KDYRGWKHWVYYTC, KDYRGWKOWVYYTC, KGWKHWVYC(NH₂) und KGWKOWVYC(NH₂).

5. Das Kammpolymer nach Anspruch 1, wobei der Polymerblock A ausgewählt ist aus der Gruppe bestehend aus Poly(ethylenglykol), Poly(propylenglykol) und Copolymeren davon.

6. Das Kammpolymer nach Anspruch 5, wobei der Polymerblock A Poly(ethylenglykol) ist, wobei der Polymerblock A eine mittlere Länge im Bereich von 3 bis 3.000 Monomereinheiten aufweist.

7. Das Kammpolymer nach Anspruch 1, wobei das Polymer die Struktur aufweist 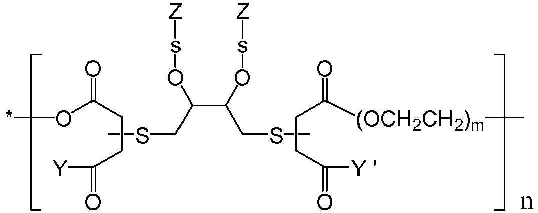 wobei m 3-3000 ist und Y und Y' unabhängig ausgewählt sind aus der Gruppe bestehend aus R, OR, COOR, SR, NHR, NRR', ONHR, NHOR, NRNH₂, NHNHR, NRNHR' und NHNRR', wobei R und R' unabhängig ausgewählt sind aus der Gruppe, bestehend aus verzweigten oder linearen C₆-C₃₀-Kohlenwasserstoffen, die gegebenenfalls mit einem oder mehreren hydrophilen Substituenten substituiert sind, cyclische oder polycyclische C₆-C₃₀-Kohlenwasserstoffe, die gegebenenfalls mit einem oder mehreren hydrophilen Substituenten substituiert sind, und hydrophobe Aminosäuren, Peptide und Polymere;
oder
wobei das Polymer die Struktur aufweist 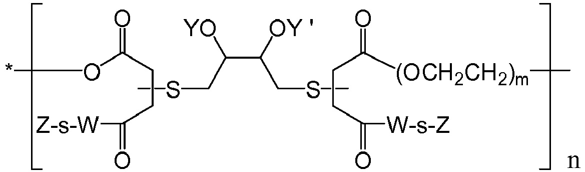 wobei m 3 bis 3.000 ist, W O oder NH ist und Y und Y' unabhängig ausgewählt sind aus der Gruppe bestehend aus R, COR, COOR, CONHR, CONRR', CONHOR, CONRNH₂, CONHNHR, CONRNHR' und CONHNRR'; wobei R und R' unabhängig ausgewählt sind aus der Gruppe, bestehend aus verzweigten oder linearen C₆-C₃₀-Kohlenwasserstoffen, die gegebenenfalls mit einem oder mehreren hydrophilen Substituenten substituiert sind, cyclische oder polycyclische C₆-C₃₀-Kohlenwasserstoffe, die gegebenenfalls mit einem oder mehreren hydrophilen Substituenten substituiert sind, und hydrophoben Aminosäuren, Peptide und Polymere;
oder
wobei das Polymer die Struktur aufweist 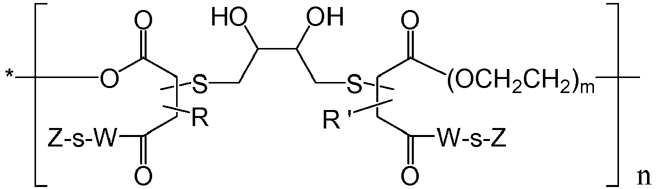 wobei m 3-3000 ist, W O oder NH ist und R und R' unabhängig ausgewählt sind aus der Gruppe, bestehend aus verzweigten oder linearen C₆-C₃₀-Kohlenwasserstoffen, die gegebenenfalls mit einem oder mehreren hydrophilen Substituenten substituiert sind, cyclische oder polycyclische C₆-C₃₀-Kohlenwasserstoffe, die gegebenenfalls mit einem oder mehreren hydrophilen Substituenten substituiert sind, und hydrophoben Aminosäuren, Peptide und Polymere.

8. Ein Peptid ausgewählt aus der Gruppe bestehend aus KGWKHWVYC(NH₂) und KGWKOWVYC(NH₂).

9. Kammpolymer nach einem der Ansprüche 1 bis 7 zur Verwendung in der Behandlung oder Prävention einer Virusinfektion.

10. Das Kammpolymer nach einem der Ansprüche 1-2 und 5 - 7 zur Verwendung in der Behandlung oder Prävention einer Influenzavirusinfektion.

## Revendications

1. Polymère en peigne consistant essentiellement en la structure suivante : 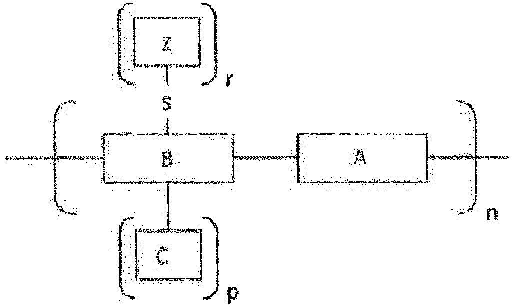 comprenant une charpente formée de fragments de point de ramification B et de blocs polymères solubles dans l'eau et hydrophiles A alternés ; et ayant des chaînes latérales hydrophobes C et des ligands optionnels Z rattachés aux fragments de point de ramification, dans lequel :
le bloc polymère soluble dans l'eau A est choisi dans l'ensemble constitué par le polyéthylèneglycol, le polypropylèneglycol, la polyéthylèneimine, le polyalcool vinylique et la polyvinylpyrrolidone, ainsi que des copolymères de ceux-ci ;
le fragment de point de ramification B est un conjugué de dithiothréitol, de dithioérythritol ou de 2,3-diaminobutane-1,4-dithiol avec deux molécules d'acide maléique ;
chaque chaîne latérale hydrophobe C a une valeur logP supérieure à 1,4, et est indépendamment choisie dans l'ensemble constitué par les hydrocarbures linéaires ou ramifiés en C₆ à C₃₀ éventuellement substitués par un ou plusieurs substituants hydrophiles ; les hydrocarbures cycliques ou polycycliques en C₆ à C₃₀ éventuellement substitués par un ou plusieurs substituants hydrophiles ; et les acides aminés hydrophobes, les peptides et les polymères ;
chaque ligand Z est indépendamment un ligand ayant une affinité de liaison spécifique pour la surface d'un virus ;
s est une liaison ou un fragment d'espacement ;
la valeur de n est située dans la plage allant de 3 à environ 100 ;
la valeur moyenne de p est située dans la plage allant de plus un à quatre ; et
la valeur moyenne de r est située dans la plage allant de 1 à 4 ;
sous réserve que le polymère en peigne ne soit pas : 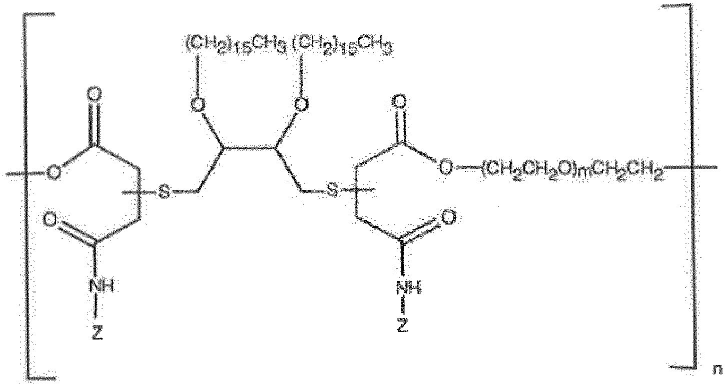 où -NH-Z est l'acide 5-acétamido-7-(6'-aminohexyl)-carbamyloxy-4-guanidino-2,3,4,5-tétradésoxy-D-glycéro-D-galacto-non-2-énopyranosonique à liaison 6'-N (zanamivir) ; 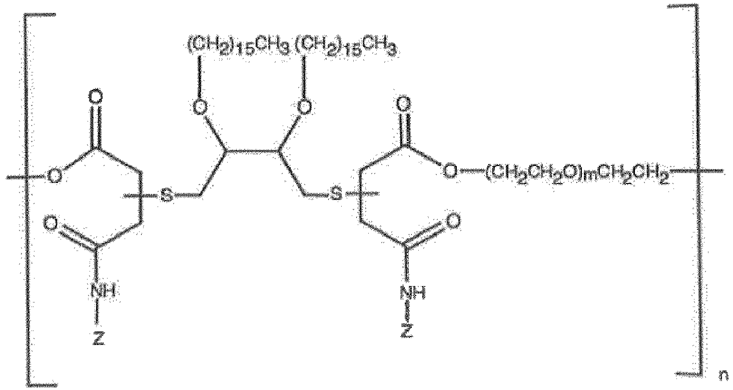 où -NH-Z est l'acide β-*O*-méthyl neuraminique à liaison N (MNA) ; ou 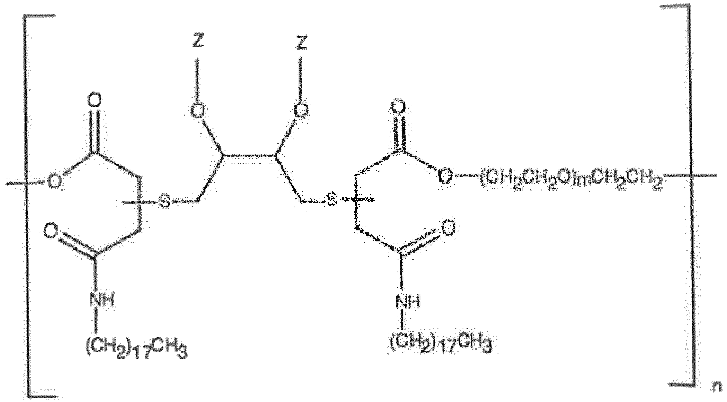 où Z est le radical neuraminyle (NANA).

2. Polymère en peigne selon la revendication 1, dans lequel au moins un ligand est l'acide N-acétylneuraminique, le β-méthylglycoside d'acide neuraminique, ou l'acide 4-guanidino-2,4-didésoxy-2,3-déshydro-N-acétylneuraminique.

3. Polymère en peigne selon la revendication 1, dans lequel au moins un ligand Z est un peptide, un anticorps ou un fragment d'anticorps ayant une affinité de liaison spécifique pour la surface dudit virus, et dans lequel le ligand est de préférence un fragment Fab ou F(ab')₂ dérivé d'immunoglobuline IgG anti-rage humaine.

4. Polymère en peigne selon la revendication 1, dans lequel au moins un ligand Z est un peptide choisi dans l'ensemble constitué par KDYRGWKHWVYYTC, KDYRGWKOWVYYTC, KGWKHWVYC (NH₂) et KGWKOWVYC(NH₂).

5. Polymère en peigne selon la revendication 1, dans lequel le bloc polymère A est choisi dans l'ensemble constitué par le polyéthylèneglycol, le polypropylèneglycol, et leurs copolymères.

6. Polymère en peigne selon la revendication 5, dans lequel le bloc polymère A est le polyéthylèneglycol, et dans lequel le bloc polymère A a une longueur moyenne située dans la plage allant de 3 à 3000 motifs monomères.

7. Polymère en peigne selon la revendication 1, lequel polymère a la structure 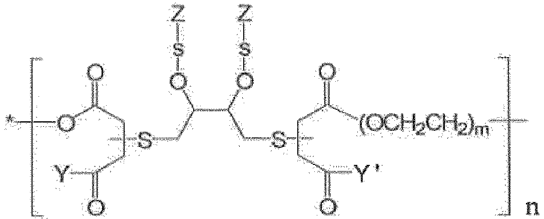 dans laquelle m vaut de 3 à 3000, et Y et Y' sont indépendamment choisis dans l'ensemble constitué par R, OR, COOR, SR, NHR, NRR', ONHR, NHOR, NRNH₂, NHNHR, NRNHR' et NHNRR' où R et R' sont indépendamment choisis dans l'ensemble constitué par les hydrocarbures linéaires ou ramifiés en C₆ à C₃₀ éventuellement substitués par un ou plusieurs substituants hydrophiles, par les hydrocarbures cycliques ou polycycliques en C₆ à C₃₀ éventuellement substitués par un ou plusieurs substituants hydrophiles, et par les acides aminés, les peptides et les polymères hydrophobes ;
ou
lequel polymère a la structure 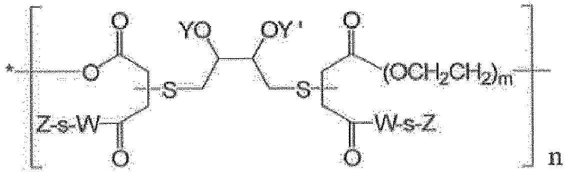 dans laquelle m vaut de 3 à 3000, W est 0 ou NH, et Y et Y' sont indépendamment choisis dans l'ensemble constitué par R, COR, COOR, CONHR, CONRR', CONHOR, CONRNH₂, CONHNHR, CONRNHR' et CONHNHRR', où R et R' sont indépendamment choisis dans l'ensemble constitué par les hydrocarbures linéaires ou ramifiés en C₆ à C₃₀ éventuellement substitués par un ou plusieurs substituants hydrophiles, les hydrocarbures cycliques ou polycycliques en C₆ à C₃₀ éventuellement substitués par un ou plusieurs substituants hydrophiles, et les acides aminés hydrophobes, les peptides et les polymères ; ou
lequel polymère a la structure 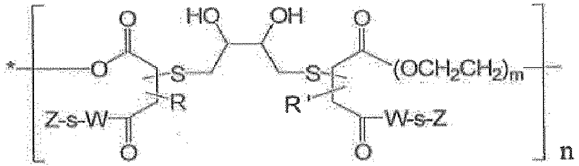 dans laquelle m vaut de 3 à 3000, W est 0 ou NH, et R et R' sont indépendamment choisis dans l'ensemble constitué par les hydrocarbures linéaires ou ramifiés en C₆ à C₃₀ éventuellement substitués par un ou plusieurs substituants hydrophiles, les hydrocarbures cycliques ou polycycliques en C₆ à C₃₀ éventuellement substitués par un ou plusieurs substituants hydrophiles, et les acides aminés hydrophobes, les peptides et les polymères.

8. Peptide choisi dans l'ensemble constitué par KGWKHWVYC(NH₂) et KGWKOWVYC(NH₂).

9. Polymère en peigne selon l'une quelconque des revendications 1 à 7, destiné à être utilisé dans le traitement ou la prévention d'une infection virale.

10. Polymère en peigne selon l'une quelconque des revendications 1, 2 et 5 à 7, destiné à être utilisé dans le traitement ou la prévention d'une infection due à un virus grippal.
